# EUROPEAN PATENT SPECIFICATION

(11) **EP 1 838 663 B1**
(45) Date of publication and mention of the grant of the patent: **21.01.2009**
(21) Application number: 05820721.8
(22) Date of filing: 21.12.2005
(51) Int. Cl.: C07C 233/78, A61K 31/166, C07C 211/27

(54) **GLYCINE TRANSPORT INHIBITORS**
INHIBITOREN DES GLYCINTRANSPORTS
INHIBITEURS DU TRANSPORT DE LA GLYCINE

(30) Priority: 23.12.2004 GB 0428231; 05.05.2005 GB 0509204; 29.11.2005 GB 0524322
(43) Date of publication of application: 03.10.2007
(73) Proprietor: GLAXO GROUP LIMITED, Greenford, Middlesex UB6 ONN (GB)
(72) Inventor: BRADLEY, Daniel Marcus, GlaxoSmithKline, Harlow Essex CM19 5AW (GB); BRANCH, Clive Leslie, GlaxoSmithKline, Harlow Essex CM19 5AW (GB); CHAN, Wai Ngor, GlaxoSmithKline, Harlow Essex CM19 5AW (GB); COULTON, Steven, GlaxoSmithKline, Harlow Essex CM19 5AW (GB); GILPIN, Martin Leonard, GlaxoSmithKline, Harlow Essex CM19 5AW (GB); HARRIS, Andrew Jonathan, GlaxoSmithKline, Ware Hertfordshire SG12 0DP (GB); LAI, Justine Yeun Quai, GlaxoSmithKline, Harlow Essex CM19 5AW (GB); MARSHALL, Howard Robert, GlaxoSmithKline, Harlow Essex CM19 5AW (GB); MACRITCHIE, Jacqueline A., Biofocus Discovery Ltd., Saffron Walden, Essex CB10 1XL (GB); NASH, David John, GlaxoSmithKline, Harlow Essex CM19 5AW (GB); PORTER, Roderick Alan, GlaxoSmithKline, Harlow Essex CM19 5AW (GB); SPADA, Simone, GlaxoSmithKline SpA, I-31735 Verona (IT); THEWLIS, Kevin Michael, GlaxoSmithKline, Harlow Essex CM19 5AW (GB); WARD, Simon Edward, GlaxoSmithKline, Harlow Essex CM19 5AW (GB)
(74) Representative: Kondo, Rie
(86) International application number: PCT/GB2005/004943
(87) International publication number: WO 2006/067417

(56) References cited:
- WO-A-03/055478
- US-A- 3 145 209

## Description

The present invention relates to glycine transporter inhibiting compounds, their use in the manufacture of medicaments for treating neurological and neuropsychiatric disorders, in particular psychoses, dementia or attention deficit disorder. The invention further comprises processes to make these compounds and pharmaceutical formulations thereof.

Molecular cloning has revealed the existence in mammalian brains of two classes of glycine transporters, termed GlyT1 and GlyT2. GlyT1 is found predominantly in the forebrain and its distribution corresponds to that of glutamatergic pathways and NMDA receptors (Smith, et al., Neuron, 8, 1992: 927-935). Molecular cloning has further revealed the existence of three variants of GlyT1, termed GlyT-1a, GlyT-1b and GlyT-1c (Kim et al., Molecular Pharmacology, 45, 1994: 608-617), each of which displays a unique distribution in the brain and peripheral tissues. The variants arise by differential splicing and exon usage, and differ in their N-terminal regions. GlyT2, in contrast, is found predominantly in the brain stem and spinal cord, and its distribution corresponds closely to that of strychnine-sensitive glycine receptors (Liu et al., J. Biological Chemistry, 268, 1993: 22802-22808; Jursky and Nelson, J. Neurochemistry, 64, 1995 : 1026-1033). Another distinguishing feature of glycine transport mediated by GlyT2 is that it is not inhibited by sarcosine as is the case for glycine transport mediated by GlyT1. These data are consistent with the view that, by regulating the synaptic levels of glycine, GlyT1 and GlyT2 selectively influence the activity of NMDA receptors and strychnine-sensitive glycine receptors, respectively.

NMDA receptors are critically involved in memory and learning (Rison and Staunton, Neurosci. Biobehav. Rev., 19 533-552 (1995); Danysz et al, Behavioral Pharmacol., 6 455-474 (1995)); and, furthermore, decreased function of NMDA-mediated neurotransmission appears to underlie, or contribute to, the symptoms of schizophrenia (Olney and Farber, Archives General Psychiatry, 52, 998-1007 (1996). Thus, agents that inhibit GlyT1 and thereby increase glycine activation of NMDA receptors can be used as novel antipsychotics and anti-dementia agents, and to treat other diseases in which cognitive processes are impaired, such as attention deficit disorders and organic brain syndromes. Conversely, over-activation of NMDA receptors has been implicated in a number of disease states, in particular the neuronal death associated with stroke and possibly neurodegenerative diseases, such as Alzheimer's disease, multi-infarct dementia, AIDS dementia, Huntington's disease, Parkinson's disease, amyotrophic lateral sclerosis or other conditions in which neuronal cell death occurs, such as stroke or head trauma. Coyle & Puttfarcken, Science, 262, 689-695 (1993); Lipton and Rosenberg, New Engl. J. of Medicine, 330, 613-622 (1993); Choi, Neuron, 1, 623-634 (1988). Thus, pharmacological agents that increase the activity of GlyT1 will result in decreased glycine-activation of NMDA receptors, which activity can be used to treat these and related disease states. Similarly, drugs that directly block the glycine site of the NMDA receptors can be used to treat these and related disease states.
Glycine transport inhibitors are already known in the art, for example as disclosed in published international patent application WO03/055478 (SmithKline Beecham).

However, there still remains the need to identify further compounds that can inhibit GlyT1 transporters, including those that inhibit GlyT1 transporters selectively over GlyT2 transporters.

It has now been found that a novel class of compounds inhibit GlyT1 transporters and are thus useful in the treatment of certain neurological and neuropsychiatric disorders, including schizophrenia.

Thus, in a first aspect, there is provided a compound of formula (I) or a salt or solvate thereof: 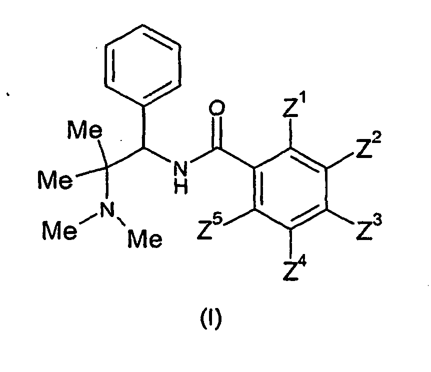 wherein
Z¹ is selected from the group consisting of C₁₋₄alkyl, C₃₋₆cycloalkyl, C₁₋₄alkylthio, haloC₁₋₄alkyl, phenyl, halophenyl, C₁-₄alkylsulfoxy, C₁₋₄alkylsulfonyl, chloro, bromo or iodo;
Z² is selected from the group consisting of hydrogen, halogen, C₁₋₄alkyl, phenyl, haloC₁₋₄alkyl, haloC₁₋₄alkoxy, halophenyl, C₁₋₄alkoxyC₁₋₄alkyl and C₃₋₆cycloalkyl;
Z³ is selected from the group consisting of hydrogen, halogen, C₁₋₄alkyl, C₁₋₄alkoxy, C₁₋₄alkylthio, haloC₁₋₄alkyl, haloC₁₋₄alkoxy, and C₃₋₆cycloalkyl;
Z⁴ is selected from the group consisting of hydrogen, halogen, C₁₋₄alkyl, haloC₁₋₄alkyl, C₁₋₄alkoxy, C₁₋₄alkylthio, phenyl, haloC₁₋₄alkoxy, halophenyl, C₁₋₄alkoxyC₁₋₄alkyl and C₃₋₆cycloalkyl;
Z⁵ is selected from the group consisting, of hydrogen, chloro, bromo, iodo, hydroxy, C₁₋₄alkyl, C₁₋₄alkoxy, C₁₋₄alkylthio, phenyl, haloC₁₋₄alkoxy, halophenyl, C₁₋₄alkoxyC₁₋₄alkyl and C₃₋₆cycloalkyl;

As used herein, the term "alkyl" refers to a straight or branched alkyl group in all isomeric forms. Examples of C₁₋₄alkyl include methyl, ethyl, propyl, isopropyl, butyl, isobutyl, sec-butyl and tert-butyl.

As used herein, the term "cycloalkyl" refers to a non-aromatic cyclic saturated hydrocarbon ring. Examples of C₃₋₆cycloalkyl include cyclopropyl, cyclobutyl, cyclopentyl, and cyclohexyl.

As used herein, the term "alkoxy" refers to the group -O-alkyl wherein alkyl is as defined above.

As used herein, the term "alkylthio" refers to the group -S-alkyl wherein alkyl is as defined above.

As used herein, the term "alkylsulfoxy" refers to the group -S(O)-alkyl wherein alkyl is as defined above.

As used herein, the term "alkysulfonyl" refers to the group -S(O)₂-alkyl wherein alkyl is as defined above.

As used herein, the terms "halogen" and its abbreviation "hal" refer to fluorine, chlorine, bromine, or iodine.

As used herein, the term "haloalkyl" refers to an alkyl group as defined above which is substituted with any number of fluorine, chlorine, bromine, or iodine atoms, including with mixtures of those atoms. A haloalkyl group may, for example contain 1, 2 or 3 halogen atoms. For example, a haloalkyl group may have all hydrogen atoms replaced with halogen atoms. Examples of haloalkyl groups include fluoromethyl, difluoromethyl and trifluoromethyl.

As used herein, the term "salt" refers to any salt of a compound according to the present invention prepared from an inorganic or organic acid or base, quaternary ammonium salts and internally formed salts. Physiologically acceptable salts are particularly suitable for medical applications because of their greater aqueous solubility relative to the parent compounds. Such salts must clearly have a physiologically acceptable anion or cation. Suitably physiologically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts. Salts having a non-physiologically acceptable anion or cation are within the scope of the invention as useful intermediates for the preparation of physiologically acceptable salts and/or for use in non-therapeutic, for example, *in vitro,* situations.

As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include water, ethanol and acetic acid. Most preferably the solvent used is water.

In one embodiment of the invention,
Z¹ is selected from the group consisting of iodo, bromo, chloro, C₁₋₄alkyl, haloC₁₋₄alkyl, C₁₋₄alkylthio, phenyl, and halophenyl;
Z² is selected from the group consisting of hydrogen, iodo, bromo, chloro, fluoro, C₁₋₄alkyl, haloC₁₋₄alkyl, haloC₁₋₄alkoxy, phenyl, and halophenyl;
Z³ is selected from the group consisting of hydrogen, iodo, bromo, chloro, C₁₋₄alkyl, haloC₁₋₄alkyl, C₁₋₄alkoxy and haloC₁₋₄alkoxy;
Z⁴ is selected from the group consisting of hydrogen, iodo, bromo, chloro, fluoro, C₁₋₄alkoxy, haloC₁₋₄alkoxy and phenyl; and
Z⁵ is selected from the group consisting of hydrogen, iodo, bromo, chloro, C₁₋₄alkyl, C₁₋₄alkoxy, haloC₁₋₄alkoxy, phenyl, and halophenyl;
wherein no more than three of Z¹, Z², Z³, Z⁴, and Z⁵ are hydrogen; wherein when Z³ is C₁₋₄alkyl, at least one of Z¹ and Z⁵ is not hydrogen; and wherein when Z⁵ is C₁₋₄alkoxy Z¹ is not hydrogen.

In another embodiment,
Z¹ is selected from the group consisting of chloro, bromo, iodo, haloC₁₋₄alkyl, phenyl, and halophenyl, and Z², Z³, Z⁴ and Z⁵ are hydrogen;

In a further embodiment,
Z¹ is selected from the group consisting of chloro and C₁₋₄alkyl;
Z² is selected from the group consisting of hydrogen, haloC₁₋₄alkyl, and C₁₋₄alkyl;
Z³ is hydrogen;
Z⁴ is hydrogen; and
Z⁵ is selected from the group consisting of hydrogen, and C₁₋₄alkyl;
wherein no more than three of Z¹, Z², Z³, Z⁴, and Z⁵ are hydrogen.

In one embodiment, Z¹ is selected from the group consisting of C₁₋₄alkyl, C₃₋₆cycloalkyl, C₁₋₂alkoxy, C₁₋₄alkylthio, haloC₁₋₄alkyl, phenyl, haloC₁₋₄alkoxy, halophenyl, C₁₋₄alkylsulfoxy, C₁₋₄alkylsulfonyl, iodo, bromo and chloro.

In one embodiment, Z¹ is selected from the group consisting of C₁₋₄alkyl, C₁₋₄alkylthio, haloC₁₋₄alkyl, C₁₋₄alkylsulfoxy, chloro and bromo. For example, Z¹ is selected from the group consisting of C₁₋₄alkylthio, C₁₋₄alkyl and haloC₁₋₄alkyl, particularly from the group consisting of methyl, methylthio and trifluoromethyl.

In one embodiment, Z² is selected from the group consisting of hydrogen, halogen, C₁₋₄alkyl and haloC₁₋₄alkyl. For example, Z² may be selected from the group consisting of hydrogen, chloro, bromo, fluoro, C₁₋₄alkyl and halo C₁₋₄alkyl. For example Z² may be hydrogen

In one embodiment, Z³ is selected from the group consisting of hydrogen, halogen, C₁₋₄alkyl, C₁₋₄alkoxy and haloC₁₋₄alkyl. For example, Z³ may be selected from the group consisting of hydrogen, chloro, fluoro, C₁₋₄alkyl and haloC₁₋₄alkyl. For example Z³ is hydrogen, chloro or trifluoromethyl.

In one embodiment, Z⁴ is selected from the group consisting of hydrogen, halogen, C₁₋₄alkyl, C₁₋₄alkoxy and haloC₁₋₄alkyl. For example Z⁴ may be selected from the group consisting of hydrogen and halogen, particularly hydrogen, fluoro and bromo. For example Z⁴ may be hydrogen.

In one embodiment, Z⁵ is selected from the group consisting of hydrogen, chloro, C₁₋₄alkyl and C₁₋₄alkoxy; Z⁵ may be selected from the group consisting of C₁₋₄alkyl and, C₁₋₄alkoxy. For example, Z⁵ may be selected from the group consisting of,methyl and methoxy.

In one embodiment, Z¹ and Z⁵ are both simultaneously not hydrogen. In a further embodiment, Z¹, Z³ and Z⁵ are all simultaneously not hydrogen.

Accordingly, in one embodiment, the present invention provides a compound of formula (Ia) or a salt or solvate thereof: 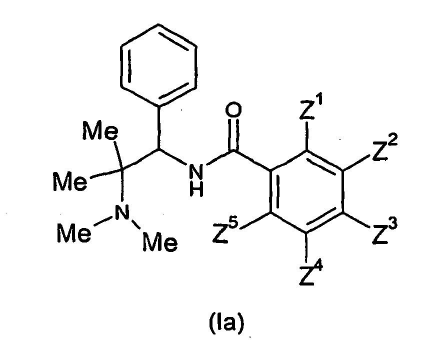 wherein
Z¹ is selected the group consisting of C₁-₄alkyl, C₁₋₄alkylthio, haloC₁₋₄alkyl, C₁₋₄alkylsulfoxy, chloro and bromo;
Z² is selected from the group consisting of hydrogen, halogen, C₁₋₄alkyl and haloC₁₋₄alkyl;
Z³ is selected from the group consisting of hydrogen, halogen, C₁₋₄alkyl, C₁₋₄alkoxy and haloC₁₋₄alkyl;
Z⁴ is selected from the group consisting of hydrogen, halogen, C₁₋₄alkyl, C₁₋₄alkoxy and haloC₁₋₄alkyl;
Z⁵ is selected from the group consisting of hydrogen, chloro, C₁₋₄alkyl and C₁₋₄alkoxy.

It is to be understood that features of an embodiment of the invention described with reference to one parameter can be combined with the features of another embodiment. The disclosure herein thus includes the combination of the features of any one embodiment with the features of any other embodiment described. All embodiments and features of compounds of formula (I) apply to compounds of formula (Ia).

Examples of compounds of the invention include Examples 1 to 65 shown below, as well as salts and solvates thereof.

The compounds of formula (I) may have the ability to crystallise in more than one form. This is a characteristic known as polymorphism, and it is understood that such polymorphic forms ("polymorphs") are within the scope of formula (I). Polymorphism generally can occur as a response to changes in temperature or pressure or both and can also result from variations in the crystallisation process. Polymorphs can be distinguished by various physical characteristics known in the art such as x-ray diffraction patterns, solubility, and melting point.

Certain of the compounds described herein may exist in stereoisomeric forms (i.e. they may contain one or more asymmetric carbon atoms or may exhibit *cis-trans* isomerism). The individual stereoisomers (enantiomers and diastereoisomers) and mixtures of these are included within the scope of the present invention. Likewise, it is understood that compounds of formula (I) may exist in tautomeric forms other than that shown in the formula and these are also included within the scope of the present invention.

As referred to above, individual enantiomers of compounds of formula (I) may be prepared. In a preferred embodiment, an optically pure enantiomer is desired. The term "optically pure enantiomer" means that the compound contains greater than about 90 % of the desired isomer by weight, preferably greater than about 95 % of the desired isomer by weight, and most preferably greater than about 99 % of the desired isomer by weight, said weight percent based upon the total weight of the isomer(s) of the compound. In some cases, one enantiomer of a particular structure may have a significantly higher activity than the other enantiomer of the same structure. Chirally pure, or chirally enriched compounds may be prepared by chirally selective synthesis or by separation of enantiomers. The separation of enantiomers may be carried out on the final product or, alternatively on a suitable intermediate. In one embodiment, the compounds of the invention are compounds of formula (I) prepared from intermediate amine (II) as defined below with stereochemistry that gives rise to (+) optical rotation in amine (II), that is to say (+)-(2-amino-1,1-dimethyl-2-phenylethyl)dimethylamine.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the working Examples.

Compounds of general formula (I) may be prepared by methods known in the art of organic synthesis as set forth in part by the following synthesis schemes. It is also recognised that in all of the schemes described below, it is well understood that protecting groups for sensitive or reactive groups are employed where necessary in accordance with general principles of chemistry. Protecting groups are manipulated according to standard methods of organic synthesis (T. W. Greene and P. G. M. Wuts (1991) Protecting Groups in Organic Synthesis, John Wiley & Sons). These groups are removed at a convenient stage of the compound synthesis using methods that are readily apparent to those skilled in the art. The selection of processes as well as the reaction conditions and order of their execution shall be consistent with the preparation of compounds of formula (I). Those skilled in the art will recognise if a stereocentre exists in compounds of formula (I). Accordingly, the present invention includes both possible stereoisomers and includes not only racemic compounds but the individual enantiomers as well. Where the stereochemistry is indicated as being variable at certain positions, a mixture of stereoisomers may be obtained, this mixture having been separated where indicated. Stereoisomers may be separated by high-performance liquid chromatography or other appropriate means. When a compound is desired as a single enantiomer, it may be obtained by stereospecific synthesis or by resolution of the final product or any convenient intermediate. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994).

Typical reaction routes for the preparation of a compound of formula (I) as hereinbefore defined, are shown in the following schemes. The starting materials and reagents are known to the skilled person in the art and/or can be prepared using methods known in the art.

Compounds of formula (I) can be synthesised by known methods; for example by, but not limited to, the synthetic route outlined in the scheme below 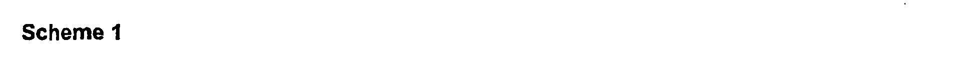 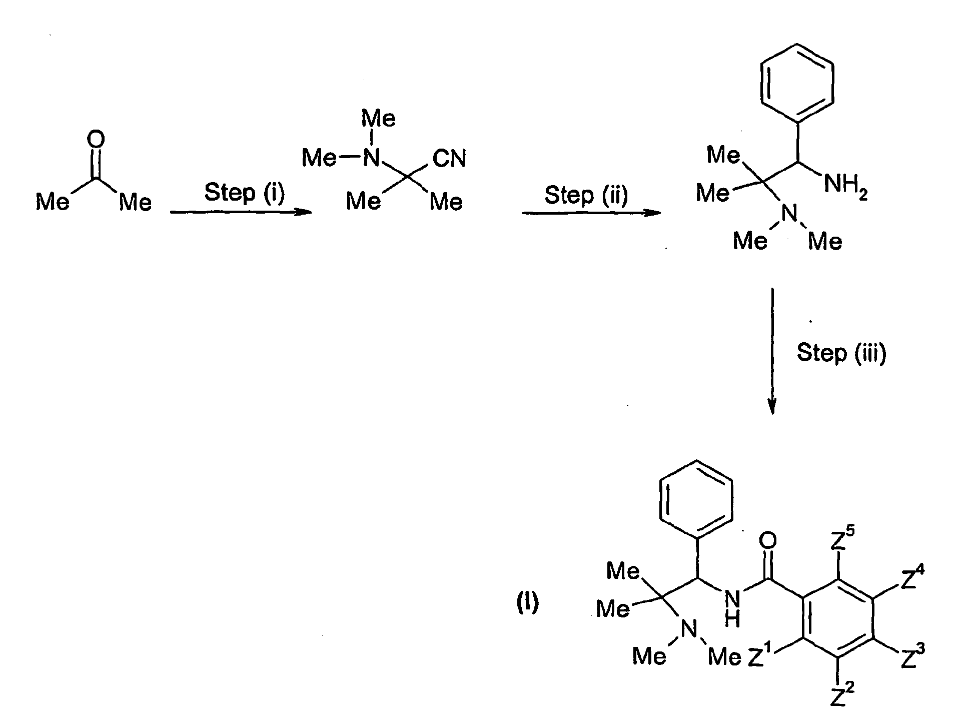 wherein Z¹, Z², Z³, Z⁴ and Z⁵ are as defined for the compound of formula (I).

Step (i) is carried out for example by reaction of acetone with an amine or amine salt in the presence of inorganic cyanide, for example potassium cyanide, in solvent such as water or by reaction of acetone with an amine and trimethylsilyl cyanide in either the absence of solvent or in a solvent such as acetic acid.

Step (ii) can be achieved by successive reaction with an appropriate organometallic reagent, for example phenyllithium, in a suitable inert solvent for example tetrahydrofuran, followed by reduction with a reducing agent, for example, sodium borohydride in a suitable solvent, for example methanol.

Acylation step (iii) can be achieved by reaction with a compound of formula (III): 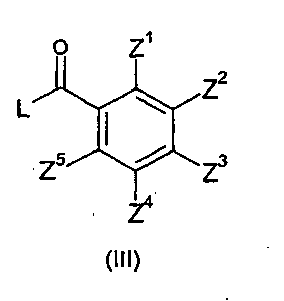 wherein Z¹, Z², Z³, Z⁴ and Z⁵ are as defined in formula (I) and L represents a suitable leaving group. Examples of leaving groups include halogen, hydroxy, OC(=O)alkyl, OC(=O)O-alkyl and OSO₂Me. L may be halogen and acylation in step (iii) may be carried out in an inert solvent such as dichloromethane, in the presence of a base such as triethylamine. When L represents hydroxy, the reaction preferably takes place in an inert solvent such as dichloromethane in the presence of a coupling reagent, for example a diimide reagent such as N,N dicyclohexylcarbodiimide (DCC), N-(3-(dimethylamino)propyl)-N-ethylcarbodiimide hydrochloride (EDC), polymer-supported EDC, polymer-supported DCC or O-(7-azabenzotriazol-1-yl)-1,1,3,3-tetramethyluronium hexafluoro phosphate (HATU). For example the coupling reagent may be selected from a diimide reagent such as N,N dicyclohexylcarbodiimide (DCC), N-(3-(dimethylamino)propyl)-N-ethylcarbodiimide hydrochloride (EDC), polymer-supported EDC or polymer-supported DCC.

Within the scheme there is scope to convert a group Z¹ into another group Z¹ and similarly for groups Z², Z³, Z⁴ and Z⁵.

Accordingly, in a second aspect, the present invention provides a method of preparing a compound of formula (I), comprising the step of:
reacting a compound of formula (II): 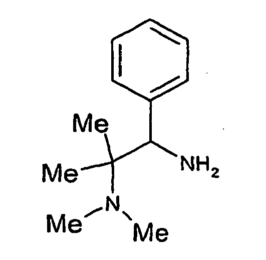 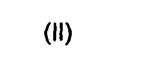
with a compound of formula (III): 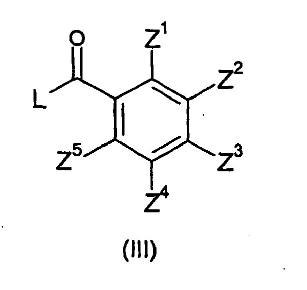
wherein Z¹, Z², Z³, Z⁴ and Z⁵ are as defined in formula (I) and L represents a suitable leaving group;
and thereafter optionally:
- removing any protecting groups and/or
- converting a compound of formula (I) into another compound of formula (I) and/or
- forming a salt or solvate.

Suitable leaving groups L include halogen, hydroxy, OC(=O)alkyl, OC(=O)O-alkyl and OSO₂Me.

Compounds of formula (I) can be converted into further compounds of formula (I) using standard techniques. For example, and by way of illustration rather than limitation, possible conversion reactions include acylation with an appropriate acylating agent such as acetyl chloride, alkylation using an appropriate alkylating reagent such as methyl iodide, and sulfonylation using a sulfonylating agent such as methanesulfonic anhydride and N-alkylation by reductive amination using a ketone or an aldehyde in the presence of a reducing agent such as sodiumtriacetoxy.

Pharmaceutically acceptable salts may be prepared conventionally by reaction with the appropriate acid or acid derivative.

In a further aspect, the present invention provides a compound of formula (II):

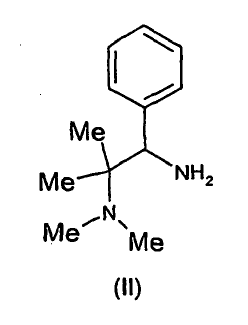

Compounds of formula (II) are useful as intermediates in the synthesis of compounds of the invention.

The compounds of the present invention inhibit the GlyT1 transporter. The compounds may selectively inhibit the GlyT1 transporter over the GlyT2 transporter.

Such compounds would be suitable for the treatment of certain neurological and neuropsychiatric disorders. As used herein, the terms "treatment" and "treating" refer to the alleviation and/or cure of established symptoms as well as prophylaxis.

The affinities of the compounds of this invention for the GlyT1 transporter can be determined by the following assay:
HEK293 cells expressing the Glycine (Type 1) transporter were grown in cell culture medium [DMEM/NUT mix F12 containing 2mM L-Glutamine, 0.8mg/mL G418 and 10% heat inactivated fetal calf serum] at 37°C and 5% CO₂. Cells grown to 70-80% confluency in T175 flasks were harvested and resuspended at 1.32x10⁶ cells/mL in assay buffer [140mM NaCl, 5.4mM KCI, 1.8mM CaCl₂, 0.8mM MgSO₄, 20mM HEPES, 5mM glucose and 5mM alanine, pH 7.4]. Compounds were serially diluted 2.5-fold in DMSO from a top concentration of 2.5mM with each compound giving a 11 data point dose-response. 100nL of compound at each concentration was added to the assay plate. An equal volume of Leadseeker^{™} WGA SPA beads (12.5mg/ml suspended in assay buffer) was added to the cell suspension (1.32 x 10⁶⁾ and 5µL of the cell/bead suspension transferred to each well of a 384-well white solid bottom plate (3300 cells/well) containing 100nL of test compounds. Substrate (5µL) was added to each well [1:100 dilution of [³H]-glycine stock in assay buffer containing 2.5µM glycine). Final DMSO concentration was 1% v/v.

Data was collected using a Perkin Elmer Viewlux. plC₅₀ values were determined using ActivityBase.

### The following assay may also be used:

HEK293 cells expressing the Glycine (Type 1) transporter are grown in cell medium (DMEM/NUT mix F12) containing 2 mM L-Glutamine, 0.8 mg/mL G418 and 10% heat inactivated fetal calf serum (Gibco BRL) at 37°C in 5% CO₂. Cells grown to 70-80% confluency in T175 flasks are harvested and resuspended at 4x10⁵ cells/ml in assay buffer [NaCl (140 mM), KCI (5.4 mM), CaCl₂ (1.8 mM), MgSO₄ (0.8 mM), HEPES (20mM), glucose (5 mM) and alanine (5 mM), pH 7.4]. An equal volume of Leadseeker^{™} SPA beads (12.5mg/ml suspended in assay buffer) is added to the cell suspension. Compounds are prepared as 10mM stocks in DMSO. 2.5 fold serial dilutions of the compounds are made in DMSO from a top conc of 2.5 mM. 100 nL of compound at each concentration is added to the assay plate (384-well white solid bottom plate) using the hummingbird dispenser. 5uL of the cell/bead mix is then added on top of the compound using a multidrop dispenser. Substrate (5uL) is then added to each well (1:100 dilution of H3-glycine in assay buffer containing 2.5 uM glycine) Data is collected using a PerkinElmer Viewlux as 5 minute exposures. plC50 data values are determined using Activity Base.

Compounds may be assayed in their free base form or in the form of a salt, for example the hydrochloride salt or the formate salt. The assays described above are generally considered to provide data that is correct to ±3 standard deviations = ±0.5.

Compounds having a pIC₅₀ at the GlyT1 transporter of greater than or equal to 5.0 are considered to be active at the GlyT1 transporter. The example compounds below were found to have a _{P}IC₅₀ at the GlyT1 transporter of greater than or equal to 5.0.

Accordingly, in a further aspect of the invention, there is provided a compound of formula (I) or a salt or solvate thereof: for use in therapy.

In another aspect of the invention, there is provided a compound of formula (I) as hereinbefore described or a salt or solvate thereof, for use in the treatment of a disorder mediated by GlyT1.

As used herein, the term "a disorder mediated by GlyT1" refers to a disorder that may be treated by the administration of a medicament that alters the activity of the GlyT1 transporter. As hereinbefore described, the action of GlyT1 transporters affects the local concentration of glycine around NMDA receptors. As a certain amount of glycine is needed for the efficient functioning of NMDA receptors, any change to that local concentration can affect NMDA-mediated neurotransmission. As hereinbefore described, changes in NMDA-mediated neurotransmission have been implicated in certain neuropsychiatric disorders such as dementia, depression and psychoses, for example schizophrenia, and learning and memory disorders, for example attention deficit disorders and autism. Thus, alterations in the activity of the GlyT1 transporter are expected to influence such disorders.

The disorders mediated by GlyT1 referred to herein include neurological and neuropsychiatric disorders, including psychoses such as schizophrenia, dementia and other forms of impaired cognition such as attention deficit disorders and organic brain syndromes. Other neuropsychiatric disorders include drug-induced (phencyclidine, ketamine and other dissociative anesthetics, amphetamine and other psychostimulants and cocaine) psychosis, psychosis associated with affective disorders, brief reactive psychosis, schizoaffective psychosis, and psychosis NOS, "schizophrenia-spectrum" disorders such as schizoid or schizotypal personality disorders, or illness associated with psychosis (such as major depression, manic depressive (bipolar) disorder, Alzheimer's disease and post-traumatic stress syndrome), and NMDA receptor-related disorders such as autism, depression, benign forgetfulness, childhood learning disorders and closed head injury.

The compounds of formula (I) are of use as antipsychotic agents for example in the treatment of schizophrenia, schizo-affective disorders, schizophreniform diseases, psychotic depression, mania, acute mania, paranoid and delusional disorders.

Within the context of the present invention, the terms used herein are classified in the Diagnostic and Statistical Manual of Mental Disorders, 4^{th} Edition, published by the American Psychiatric Association (DSM-IV) and/or the International Classification of Diseases, 10^{th} Edition (ICD-10). The various subtypes of the disorders mentioned herein are contemplated as part of the present invention. Numbers in brackets after the listed diseases below refer to the classification code in DSM-IV.

In particular, the compounds of formula (I) are of use in the treatment of schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and 'With Hallucinations; Substance-Induced Psychotic Disorder including the subtypes With Delusions (293.81) and With Hallucinations (293.82); and Psychotic Disorder Not Otherwise Specified (298.9).

The compounds of formula (I) are also of use in the treatment of mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90).

The compounds of formula (I) are also of use in the treatment of anxiety disorders including Panic Attack, Agoraphobia, Panic Disorder, Agoraphobia Without History of Panic Disorder (300.22), Specific Phobia (300.29) including the subtypes Animal Type, Natural Environment Type, Blood-Injection-Injury Type, Situational Type and Other Type), Social Phobia (300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder and Anxiety Disorder Not Otherwise Specified (300.00).

The compounds of formula (I) are also of use in the treatment of substance-related disorders including Substance Use Disorders such as Substance Dependence and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide.

The compounds of formula (I) are also of use in the treatment of sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition; and Substance-Induced Sleep Disorder including the subtypes Insomnia Type, Hypersomnia Type, Parasomnia Type and Mixed Type.

The compounds of formula (I) are also of use in the treatment of eating disorders such as Anorexia Nervosa (307.1) including the subtypes Restricting Type and Binge-Eating/Purging Type; Bulimia Nervosa (307.51) including the subtypes Purging Type and Nonpurging Type; Obesity; Compulsive Eating Disorder, and Eating Disorder Not Otherwise Specified (307.50).

The compounds of formula (I) are also of use in the treatment of Autistic Disorder (299.00); Attention-Deficit /Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23).

The compounds of formula (I) are also of use in the treatment of Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9).

The compounds of Formula (I) are also of use in the enhancement of cognition including the treatment of cognition impairment in other diseases such as schizophrenia, bipolar disorder, depression, other psychiatric disorders and psychotic conditions associated with cognitive impairment. Within the context of the present invention, the term cognitive impairment includes for example the treatment of impairment of cognitive functions including attention, orientation, learning disorders, memory (i.e. memory disorders, amnesia, amnesic disorders, transient global amnesia syndrome and age-associated memory impairment) and language function; cognitive impairment as a result of stroke, Alzheimer's disease, Huntington's disease, Pick disease, Aids-related dementia or other dementia states such as Multiinfarct dementia, alcoholic dementia, hypotiroidism-related dementia, and dementia associated to other degenerative disorders such as cerebellar atrophy and amyotropic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, mild cognitive impairment, age related cognitive impairment, autism related cognitive impairment, Down's syndrome, cognitive deficit related to psychosis, and post-electroconvulsive treatment related cognitive disorders; and dyskinetic disorders such as Parkinson's disease, neuroleptic-induced parkinsonism, and tardive dyskinesias.

The compounds of formula (I) are also of use in the treatment of sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9).

The invention also provides a compound of formula (I) as hereinbefore described or a pharmaceutically acceptable salt or solvate thereof for use in the treatment of schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, Parkinson's disease, dyskinetic disorders, depression, bipolar disorder, cognitive impairment, obesity, emesis, movement disorders, obsessive-compulsive disorders, amnesia, aggression, vertigo, dementia and circadian rhythm disorders.

The invention also provides a compound of formula (I) as hereinbefore described or a pharmaceutically acceptable salt or solvate thereof for use In the treatment of psychotic disorders, substance abuse, cognitive impairment, obesity, and gastric motility disorders.

The compounds of formula (I) are also of use as anticonvulsants. The compounds of formula (I) are thus useful in the treatment of convulsions in mammals, and particularly epilepsy in humans. "Epilepsy" is intended to include the following seizures: simple partial seizures, complex partial seizures, secondary generalised seizures, generalised seizures including absence seizures, myoclonic seizures, clonic seizures, tonic seizures, tonic clonic seizures and atonic seizures. The invention may also be used in a method of treating convulsions, which comprises administering to a mammal in need thereof an effective amount of a compound of formula (I) as hereinbefore described or a pharmaceutically acceptable salt or solvate thereof. Treatment of epilepsy may be carried out by the administration of a non-toxic anticonvulsant effective amount of a compound of the formula (III) or a pharmaceutically acceptable salt, or a composition as hereinbefore defined.

The compounds of formula (I) also find use in the treatment of neuropathic pain, for example in diabetic neuropathy, sciatica, non-specific lower back pain, multiple sclerosis pain, fibromyalgia, HIV-related neuropathy, neuralgia such as post-herpetic neuralgia and trigeminal neuralgia and pain resulting from physical trauma, amputation, cancer, toxins or chronic inflammatory conditions.

In another aspect of the invention, there is provided use of a compound of formula (I) as hereinbefore defined or a salt or solvate thereof in the preparation of a medicament for the treatment of a disorder mediated by GlyT1.

Preferably, the disorder mediated by GlyT1 to be treated by the use or method as hereinbefore described is a psychosis, including schizophrenia, dementia and attention deficit disorders, particularly schizophrenia.

The invention also provides the use of a compound of formula (I) as hereinbefore described or a pharmaceutically acceptable salt or solvate thereof in the manufacture of a medicament for the treatment of schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, Parkinson's disease, dyskinetic disorders, depression, bipolar disorder, cognitive impairment, obesity, emesis, movement disorders, obsessive-compulsive disorders, amnesia, aggression, vertigo, dementia and circadian rhythm disorders.

The invention also provides the use of a compound of formula (I) as hereinbefore described or a pharmaceutically acceptable salt or solvate thereof in the manufacture of a medicament for the treatment of psychotic disorders, substance abuse, cognitive impairment, obesity and gastric motility disorders.

As used herein, the term "effective amount" means that amount of a drug or pharmaceutical agent that will elicit the biological or medical response of a tissue, system, animal or human that is being sought, for instance, by a researcher or clinician.

Compounds for use according to the invention may be administered as the raw material but the active ingredients are preferably provided in the form of pharmaceutical compositions.

Accordingly, in a further aspect of the invention, there is provided a pharmaceutical composition comprising a compound of formula (I) as hereinbefore described or a salt or solvate thereof, and at least one pharmaceutically acceptable carrier, diluent or excipient.

These pharmaceutical compositions may be used in the treatment of clinical conditions for which a GlyT1 inhibitor is indicated such as, for example, schizophrenia. The carrier must be pharmaceutically acceptable to the recipient and must be compatible with, i.e. not have a deleterious effect upon, the other ingredients in the composition. The carrier may be a solid or a liquid and is preferably formulated with at least one compound of formula (I) or a salt or solvate thereof as a unit dose formulation. If desired, other physiologically active ingredients may also be incorporated in the pharmaceutical compositions of the invention.

It will be appreciated by those skilled in the art that the compounds according to the invention may advantageously be used in conjunction with one or more other therapeutic agents, for instance, different antidepressant agents such as 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1 A antagonists, 5HT1 B antagonists, 5HT1D antagonists, D1 agonists, M1 agonists and/or anticonvulsant agents, as well as atypical antipsychotic drugs and cognitive enhancers.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

Suitable anticonvulsant agents which may be used in combination of the compounds of the invention include for example divalproex, carbamazepine and diazepam.

Suitable atypical antipsychotic drugs which which may be used in combination of the compounds of the invention include for example risperidone, olanzapine, ziprasidone, aripiprazole and clozapine.

It will be appreciated that the compounds of the combination or composition may be administered simultaneously (either in the same or different pharmaceutical formulations), separately or sequentially.

The compounds of formula (I) and their pharmaceutically acceptable salts and solvates thereof are also suitable for combination with other typical and atypical antipsychotics to provide improved treatment of psychotic disorders. Particular advantages associated with the combinations, uses and methods of treatment of compounds of formula (I) and their pharmaceutically acceptable salts and solvates thereof include equivalent or improved efficacy at doses of administration which are lower than those commonly used for the individual components. Improved treatments of positive symptoms and/or negative symptoms and/or cognitive symptoms of the psychotic disorder may also be observed. The combinations, uses and methods of treatment of the invention may also provide advantages in treatment of patients who fail to respond adequately or who are resistant to treatment with certain neuroleptic agents.

The combination therapies of the invention are preferably administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is.also known as add-on therapeutic administration. Any and all treatment regimes In which a patient receives separate but coterminous or overlapping therapeutic administration of the compounds of formula (I) or a pharmaceutically acceptable salt or solvate thereof and at least one neuroleptic agent are within the scope of the current invention. In one embodiment of adjunctive therapeutic administration as described herein, a patient is typically stabilised on a therapeutic administration of one or more of the of the components for a period of time and then receives administration of another component Within the scope of this invention, it is preferred that the compounds of formula (I) or a pharmaceutically acceptable salt or solvate thereof is administered as adjunctive therapeutic treatment to patients who are receiving administration of at least one neuroleptic agent, but the scope of the invention also includes the adjunctive therapeutic administration of at least one neuroleptic agent to patients who are receiving administration of compounds of formula (I) or a pharmaceutically acceptable salt or solvate thereof.

The combination therapies of the invention may also be administered simultaneously. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for simultaneous combination may be provided in the form of a kit-of-parts.

In a further aspect, the invention provides the use of compounds of formula (I) or a pharmaceutically acceptable salt or solvate thereof in the manufacture of a medicament for adjunctive therapeutic administration for the treatment of a psychotic disorder in a patient receiving therapeutic administration of at least one neuroleptic agent. The invention further provides compounds of formula (I) or a pharmaceutically acceptable salt or solvate thereof for use for adjunctive therapeutic administration for the treatment of a psychotic disorder in a patient receiving therapeutic administration of at least one neuroleptic agent.

In a further aspect, the Invention provides the use of at least one neuroleptic agent in the manufacture of a medicament for adjunctive therapeutic administration for the treatment of a psychotic disorder in a patient receiving therapeutic administration of compounds of formula (I) or a pharmaceutically acceptable salt or solvate thereof. The invention further provides at least one neuroleptic agent for adjunctive therapeutic administration for the treatment of a psychotic disorder in a patient receiving therapeutic administration of compounds of formula (I) or a pharmaceutically acceptable salt or solvate thereof.

The invention further provides the use of a combination of compounds of formula (I) or a pharmaceutically acceptable salt or solvate thereof and at least one neuroleptic agent in the manufacture of a medicament for simultaneous therapeutic administration in the treatment of a psychotic disorder. The invention further provides the use of compounds of formula (I) or a pharmaceutically acceptable salt thereof in the manufacture of a medicament for simultaneous therapeutic administration with at least one neuroleptic agent in the treatment of a psychotic disorder. The invention further provides compounds of formula (I) or a pharmaceutically acceptable salt thereof for use for simultaneous therapeutic administration with at least one neuroleptic agent in the treatment of a psychotic disorder. The invention further provides the use of at least one neuroleptic agent in the manufacture of a medicament for simultaneous therapeutic administration with compounds of formula (I) or a pharmaceutically acceptable salt thereof in the treatment of a psychotic disorder.

In further aspects, the invention provides a pharmaceutical composition comprising compounds of formula (I) or a pharmaceutically acceptable salt or solvate thereof and at least one mood stabilising or antimanic agent, the use of a pharmaceutical composition comprising compounds of formula (I) or a pharmaceutically acceptable salt or solvate thereof and at least one mood stabilising or antimanic agent in the manufacture of a medicament for the treatment of a psychotic disorder, and a pharmaceutical composition comprising compounds of formula (I) or a pharmaceutically acceptable salt or solvate thereof and at least one mood stabilising or antimanic agent for use in the treatment of a psychotic disorder.

In a further aspect, the invention provides a kit-of-parts for use in the treatment of a psychotic disorder comprising a first dosage form comprising compounds of formula (I) or a pharmaceutically acceptable salt or solvate thereof and one or more further dosage forms each comprising a neuroleptic agent for simultaneous therapeutic administration.

Within the context of the present invention, the term psychiatric disorder includes those disorders mentioned above, such as schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, dyskinetic disorders, depression, bipolar disorder, cognitive impairment and obsessive-compulsive disorders and all the various forms of the disorders as mentioned herein, which are contemplated as part of the present invention.

Examples of neuroleptic/antipsychotic drugs that are useful in the present invention include, but are not limited to: butyrophenones, such as haloperidol, pimozide, and droperidol; phenothiazines, such as chlorpromazine, thioridazine, mesoridazine, trifluoperazine, perphenazine, fluphenazine, thiflupromazine, prochlorperazine, and acetophenazine; thioxanthenes, such as thiothixene and chlorprothixene ; thienobenzodiazepines; dibenzodiazepines; benzisoxazoles; dibenzothiazepines; imidazolidinones ; benzisothiazolyl-piperazines; triazine such as lamotrigine; dibenzoxazepines, such as loxapine; dihydroindolones, such as molindone; aripiprazole; and derivatives thereof that have antipsychotic activity.

Examples of neuroleptic drugs that are preferred for use in the present invention are shown in Table A.

**Table A**

| Neuroleptic drugs | | | | |
|---|---|---|---|---|
| **Common Name** | **Trade Name** | **Route of Administration** | **Form** | **Dosage Range and (Median)^{a}** |
| Clozapine | CLOZARlL | oral | tablets | 12.5-900 mg/day (300-900 mg/day) |
| Olanzapine | ZYPREXA | oral | tablets | 5-25 mg/day (10-25 mg/day) |
| Ziprasidone | GEODON | oral | capsules | 20-80mg/twice a day (80-160 mg/day) |
| Risperidone | RISPERDAL | oral | solution tablets | 2-16 mg/day tablets (4-12 mg/day) |
| Quetiapine fumarate | SEROQUEL | oral | tablets | 50-900 mg/day (300-900 mg/day) |
| Sertindole | SERLECT | | | (4-24 mg/day) |
| Amisulpride | | | | |
| Haloperidol | HALDOL | oral | tablets | 1-100 mg/day (1-15 mg/day) |
| Haloperidol Decanoate | HALDOL Decanoate | parenteral | injection | |
| Haloperidol-lactate | HALDOL INTENSOL | oral | solution | |
| | | parenteral | injection | |
| Chlorpromazine | THORAZINE | rectal | suppositories | 30-800 mg/day (200-500 mg/day) |
| | | oral | capsules solution tablets | |
| | | parenteral | injection | |
| Fluphenazine | PROLIXIN | | | 0.5-40 mg/day (1-5 mg/day) |
| Fluphenazine decanoate | PROLIXIN Decanoate | parenteral | injection | (about one-half the dosage shown for oral) |
| Fluphenazine enanthate | PROLIXIN | parenteral | injection | (same as above |
| Fluphenazine hydrochloride | PROUXIN | oral | elixer solution | |
| | | parenteral | injection | |
| Thiothixene | NAVANE | oral | capsules | 6-60 mg/day (8-30 mg/day) |
| Thiothixene hydrochloride | NAVANE | oral | solution | |
| | | parenteral | injection | |
| Trifluoperazine | STELAZINE | | | (2-40 mg/day) |
| Perphenazine | TRILAFON | oral | solution tablets | 12-64 mg/day (16-64 mg/day) |
| | | parenteral | injection | |
| Perpehazine and Amitriptyline hydrochloride | ETRAFON TRIAVIL | oral | tablets | |
| Thioridazine | MELLARIL | oral | suspension solution tablets | 150-800 mg/day (100-300 mg/day) |
| Mesoridazine | | | | (30-400 mg/day) |
| | | | | |
| Molindone | MOBAN | | | 50-225 mg/day (15-150 mg/day) |
| Molindone hydrochloride | MOBAN | oral | solution | |
| Loxapine | LOXITANE | | | 20-250 mg/day (60-100 mg/day) |
| Loxapine hydrochloride | LOXITANE | oral | solution | |
| | | parenteral | injection | |
| Loxapine succinate | LOXITANE | oral | capsules | |
| Pimozide | | | | (1-10 mg/day) |
| Flupenthixol | | | | |
| Promazine | SPARINE | | | |
| Triflupromazine | VESPRIN | | | |
| Chlorprothixene | TARACTAN | | | |
| Droperidol | INAPSINE | | | |
| Acetophenazine | TINDAL | | | |
| Prochlorperazine | COMPAZINE | | | |
| Methotrimeprazine | NOZINAN | | | |
| Pipotiazine | PIPOTRIL | | | |
| Aripiprazole | | | | |
| Hoperidone | | | | |

Examples of tradenames and suppliers of selected neuroleptic drugs are as follows : clozapine (available under the tradename CLOZARIL®, from Mylan, Zenith Goldline, UDL, Novartis); olanzapine (available under the tradename ZYPREX®, from Lilly ; ziprasidone (available under the tradename GEODON®, from Pfizer); risperidone (available under the tradename RISPERDAL®, from Janssen); quetiapine fumarate (available under the tradename SEROQUEL®, from AstraZeneca); haloperidol (available under the tradename HALDOL®, from Ortho-McNeil); chlorpromazine (available under the tradename THORAZINE®, from SmithKline Beecham (GSK); fluphenazine (available under the tradename PROLIXIN®, from Apothecon, Copley, Schering, Teva, and American Pharmaceutical Partners, Pasadena); thiothixene (available under the tradename NAVANE®; from Pfizer); trifluoperazine (10-[3-(4-methyl-1-piperazinyl)propyl]-2-(trifluoromethyl)phenothiazine dihydrochloride, available under the tradename STELAZINE®, from Smith Klein Beckman; perphenazine (available under the tradename TRILAFON®; from Schering); thioridazine (available under the tradename MELLARIL®; from Novartis, Roxane, HiTech, Teva, and Alpharma) ; molindone (available under the tradename MOBAN®, from Endo); and loxapine (available under the tradename LOXITANE®; from Watson). Furthermore, benperidol (Glianimon®), perazine (Taxilan®) or melperone (Eunerpan®)) may be used.

Other preferred neuroleptic drugs include promazine (available under the tradename SPARINE®), triflurpromazine (available under the tradename VESPRIN®), chlorprothixene (available under the tradename TARACTAN®), droperidol (available under the tradename INAPSINE®), acetophenazine (available under the tradename TINDAL®;), prochlorperazine (available under the tradename COMPAZINE®), methotrimeprazine (available under the tradename NOZINAN®), pipotiazine (available under the tradename PIPOTRIL®), ziprasidone, and hoperidone.

Particularly preferred neuroleptic agents for use in the invention are olanzapine, risperidone, quetiapine, aripiprazole, haloperidol, clozapine, ziprasidone and osanetant.

It will be appreciated by those skilled in the art that the compounds according to the invention may advantageously be used in conjunction with one or more other therapeutic agents, for instance, different antidepressant agents such as 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists and/or anticonvulsant agents, as well as atypical antipsychotic drugs and cognitive enhancers.

Suitable 5HT3 antagonists which may be used in combination of the compounds of the inventions include for example ondansetron, granisetron, metoclopramide.
Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

Suitable anticonvulsant agents which may be used in combination of the compounds of the invention include for example divalproex, carbamazepine and diazepam.

Suitable atypical antipsychotic drugs which which may be used in combination of the compounds of the invention include for example risperidone, olanzapine, ziprasidone, aripiprazole and clozapine.

It will be appreciated that the compounds of the combination or composition may be administered simultaneously (either in the same or different pharmaceutical formulations), separately or sequentially.

For use in medicine, the compounds of the present invention are usually administered as a standard pharmaceutical composition. The present invention therefore provides in a further aspect a pharmaceutical composition comprising a compound of formula (I) as hereinbefore described or a pharmaceutically (i.e. physiologically) acceptable salt thereof and a pharmaceutically (i.e. physiologically) acceptable carrier. The pharmaceutical composition can be for use in the treatment of any of the conditions described herein.

Possible formulations include those suitable for oral, sub-lingual, buccal, parenteral (for example, subcutaneous, intramuscular, or intravenous), rectal, topical and intranasal administration and in forms suitable for administration by inhalation or insufflation (either through the mouth or nose). The most suitable means of administration for a particular patient will depend on the nature and severity of the conditions being treated and on the nature of the active compound, but, where possible, oral administration is preferred.

Formulations suitable for oral administration may be provided as discrete units, such as tablets, capsules, cachets, or lozenges, each containing a predetermined amount of the active compound; as powders or granules; as solutions or suspensions in aqueous or non-aqueous liquids; or as oil-in-water or water-in-oil emulsions. For example, a compound of the invention may be prepared as a formulation with a controlled release profile. This may be in any of the above mentioned pharmaceutical forms. For example, it may be a gel formulation in a non aqueous oily vehicle, for example Miglyol, with a suitable gelling agent if required, for example methyl cellulose or hydrophobic colloidal silica.

Formulations suitable for sublingual or buccal administration include lozenges comprising the active compound and, typically, a flavoured base, such as sugar and acacia or tragacanth and pastilles comprising the active compound in an inert base, such as gelatin and glycerin or sucrose and acacia.

Formulations suitable for parenteral administration typically comprise sterile aqueous solutions containing a predetermined concentration of the active compound; the solution is preferably isotonic with the blood of the intended recipient. Although such solutions are preferably administered intraveneously, they may also be administered by subcutaneous or intramuscular injection.

Formulations suitable for rectal administration are preferably provided as unit-dose suppositories comprising the active ingredient and one or more solid carriers forming the suppository base, for example, cocoa butter.

Formulations suitable for topical or intranasal application include ointments, creams, lotions, pastes, gels, sprays, aerosols and oils. Suitable carriers for such formulations include petroleum jelly, lanolin, polyethylene glycols, alcohols, and combinations thereof.

Formulations of compounds of the invention may, for example, be composed so as to improve the exposure profile of the compound of the invention.

Compositions suitable for transdermal administration include ointments, gels and patches. Preferably the composition is in unit dose form such as a tablet, capsule or ampoule.

The formulations of the invention may be prepared by any suitable method, typically by uniformly and intimately admixing the active compound(s) with liquids or finely divided solid carriers, or both, in the required proportions and then, if necessary, shaping the resulting mixture into the desired shape.

For example, a tablet may be prepared by compressing an intimate mixture comprising a powder or granules of the active ingredient and one or more optional ingredients, such as a binder, lubricant, inert diluent, or surface active dispersing agent, or by moulding an intimate mixture of powdered active ingredient and inert liquid diluent.

Aqueous solutions for parenteral administration are typically prepared by dissolving the active compound in sufficient water to give the desired concentration and then rendering the resulting solution sterile and isotonic.

It will be appreciated that the precise dose administered will depend on the age and condition of the patient and the frequency and route of administration and will be at the ultimate discretion of the attendant physician. The compound may be administered in single or divided doses and may be administered one or more times, for example 1 to 4 times per day.

A proposed dose of the active ingredient for use according to the invention for oral, sub-lingual, parenteral, buccal, rectal, intranasal or topical administration to a human (of approximately 70 kg bodyweight) for the treatment of neurological and neuropsychiatric disorders mediated by a GlyT1 inhibitor, including schizophrenia, may be about 1 to about 1000 mg, preferably about 5 to about 500 mg, more preferably about 10 to about 100 mg of the active ingredient per unit dose which could be administered, for example, 1 to 4 times per day.

Compounds of the invention may be used as PET ligands (for example labelled with carbon-11 or fluorine-18) or as SPECT ligands (for example labelled with iodine-123 or meta stable technetium-99) for *in vivo* visualisation and quantification of the GlyT1 transporter. For example, they may be used in PET or SPECT imaging of the brain. In the context of this patent, PET shall mean: positron emission tomography and SPECT (= SPET) shall mean: single photon emission (computed) tomography.

The invention is further illustrated by the following non-limiting examples.

### Abbreviations:

| | |
|---|---|
| THF | tetrahydrofuran |
| DCM | dichloromethane |
| DMF | dimethylformamide |
| EDC | N-(3-(dimethylamino)propyl)N-ethylcarbodiimide hydrochloride |
| HOAt | 3H-(1,2,3)-triazolo(4,5-b)pyridine-3-ol |
| NMP | N-methylpyrrolidinone |
| DIPEA | N,N-diisopropylethylamine |
| HOBt | 1-hydroxybenzotriazole hydrate |

### Analytical LC/MS chromatography conditions:

### Method A

| | |
|---|---|
| Column: | Waters Atlantis 50mm x 4.6mm, 3um particle size |
| Mobile phase: | A: 0.05% Formic acid + Water |
| | B: Acetonitrile +0.05% Formic acid |
| Gradient: | 5-min runtime: 3%B to 97%B over 4min |
| Flow rate: | 3 ml/min |
| UV wavelength range: | 220 -330 nm |
| Temperature: | 30°C |

### Method B

| | |
|---|---|
| Column: | Waters Atlantis 20mm x 4.6mm, 3um particle size |
| Mobile phase: | A: 0.1% Formic acid + Water |
| | B: Acetonitrile +0.1 % Formic acid |
| Gradient: | 5.5-min runtime: 3%B to 97%B over 5.3min |
| Flow rate: | 1 ml/min |
| UV wavelength range: | 210-350 nm |
| Temperature: | Ambient |

### Mass Directed Auto-Purification System chromatography conditions:

| | |
|---|---|
| Column: | Waters Atlantis 19mm x 100mm or 30mm X 100mm, 5um particle size |
| Mobile phase: | A: 0.1 % Formic acid + Water |
| | B: Acetonitrile +0.1% Formic acid |
| Gradient: | 13.5 min runtime with 10min gradient dependant on analytical retention time |
| Flow rate: | 20 or 40 ml/min |

### Description 1: 2-(Dimethylamino)-2-methylpropanenitrile

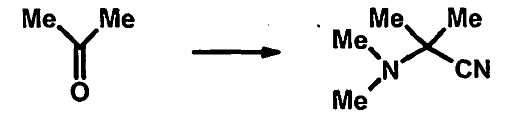

To an ice-cooled suspension of dimethylamine hydrochloride (8.15g; 0.1mol) in acetone (7.54ml; 0.1mol) was added dropwise a solution of potassium cyanide (6.51g; 0.1mol) in water (50ml) over 10min. After stirring at room temperature overnight, the crude reaction mixture was extracted twice with diethyl ether (250ml) and the combined extracts washed with saturated brine (150ml), dried (MgSO₄) and evaporated to afford the title product as a colourless liquid (8.7g, 78%) which was used without further purification. ¹H NMR (CDCl₃) δ: 1.5 (6H, s), 2.3 (6H, s).

### Description 2: (±)-(2-Amino-1,1-dimethyl-2-phenylethyl)dimethylamine

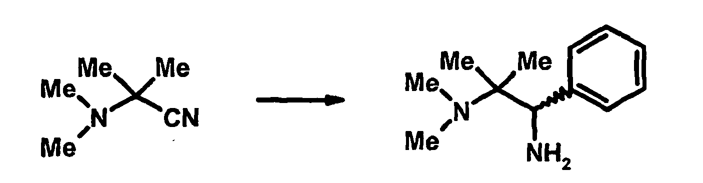

To a solution of 2-(dimethylamino)-2-methylpropanenitrile D1 (8.7g; 77.7mmol) in anhydrous THF (400ml) at -70°C under argon was added a solution of phenyllithium in dibutylether (86.3ml of a 1.8M solution; 155mmol) over 10 minutes. The reaction mixture was stirred at -70°C for 2h., cooling removed and allowed to warm to room temperature and stirred overnight. The mixture was cooled in ice and saturated aqueous sodium hydrogen carbonate (400ml) was added and the mixture was stirred for 0.5h. The layers were separated and the aqueous layer extracted with diethyl ether (200ml). Combined organics were dried (Na₂SO₄) and the solvent evaporated *in-vacuo.* The residue was dissolved in methanol (400ml) and cooled in ice as sodium borohydride (5.2g, 138mmol) was added in 4 portions over 5 minutes. After 0.5h. stirring with ice cooling, cooling was removed and stirring continued for a further 1.5h. Water (50ml) was carefully added with ice cooling and the resultant concentrated to ca.70ml *in-vacuo*, and then partitioned between 2N HCl (100ml) and ethyl acetate (400ml). The organic layer was extracted with 2N HCl (2 x 100ml) and the combined acid extracts washed with ethyl acetate (200ml), basified with 50% NaOH and extracted into DCM (3 x 150ml). Combined extracts were dried (Na₂SO₄) and evaporated *in-vacuo* to afford the title compound as a colourless oil (13g, 87%). ¹H NMR (CDCl₃) δ:0.72 (3H, s), 0.95 (3H, s), 1.78 (2H, bs), 2.29 (6H,s), 4.16 (1 H, s), 7.15 - 7.45 (5H, m).

### Description 3: (+)-(2-Amino-1,1-dimethyl-2-phenylethyl)dimethylamine

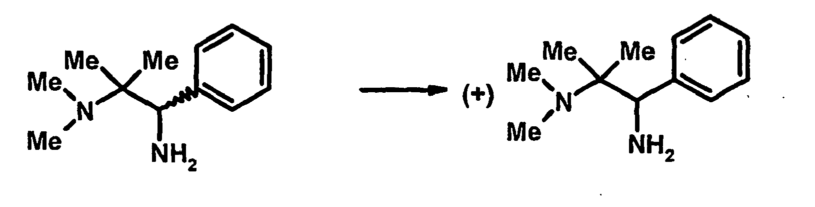

A solution of (R)-(-)-α-methoxyphenylacetic acid (10.18g, 61.25mmol) in isopropanol (120ml) was added over 5 minutes in a slow steady stream to a stirred solution of (±)-(2-amino-1,1-dimethyl-2-phenylethyl)dimethylamine D2 (11.76g, 61.25mmol) in isopropanol (236ml) at 60°C. On complete addition the colourless solution was allowed to cool gradually with stirring to room temperature. The colourless solid was filtered, washed with cold isopropanol, then diethyl ether and dried. The mother liquors were evaporated *in-vacuo* to afford a colourless solid. The solids were combined and dissolved in the minimum of boiling isopropanol (approximately 800ml). The solution was allowed to cool for 1h. and the resultant colourless solid filtered, washed with room temperature isopropanol, then diethyl ether and dried. A small sample was converted to the title product: Chiral HPLC 98.1%ee. (Supercritical Fluid Chromatography Chiralcel OD (250 mm x 4.6 mm i.d; 10 micron particle size) as the stationary phase with a mobile phase of Carbon Dioxide: Methanol (80:20) v/v; pump-mixed) at a flow-rate of 2.35 mL/min; pressure 100 bar. Chromatography performed at 38°C with detection by U.V. absorbance at 215 nm. The bulk of the salt was partitioned between 1N NaOH (100ml) and DCM (200ml) and the aqueous layer extracted with DCM (2 x 200ml). Combined organics were dried (Na₂SO₄) and evaporated to afford the title compound as a colourless oil (4.15g, 35%). ¹H NMR (CDCl₃) δ:0.72 (3H, s), 0.95 (3H, s), 1.78 (2H, bs), 2.29 (6H, s), 4.16 (1H. s), 7.15 - 7.45 (5H, m). [α]_{D}= +25.6° (25°C , c = 1, CHCl₃).

### Alternative procedure:

A solution of (R)-(-)-α-methoxyphenylacetic acid (0.874g, 5.263mmol) in isopropanol (5ml) was added, over 2 minutes dropwise to a stirred solution of (±)-(2-amino-1,1-dimethyl-2-phenylethyl)dimethylamine D2 (1g, 5.263mmol) in isopropanol (10ml) at 50°C. After 2 minutes crystals formed. Heating was then removed and the mixture was allowed to cool to room temperature and the mixture was allowed to stand for 2 hours before being diluted with 50ml ice cold isopropanol. The solid was filtered, washed with ice cold isopropanol, then diethyl ether to give 0.89g of crystals, of which 600mg was recrystallised from isopropanol, recovery 480mg. After partitioning the crystals between 1N NaOH and DCM, separating the phases by a phase separation cartridge and blowing off solvent the title compound was obtained: Chiral HPLC 99.8%ee.

### Description 4: 2-Methyl-4,6-bis(trifluoromethyl)benzoic acid

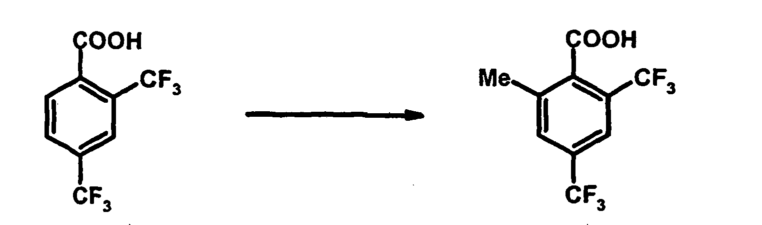

Dry THF (5ml) was stirred under argon at -80°C and treated with sec-butyl lithium (3.05ml of a 1.4M solution in cyclohexane, 4.27mmol) and N,N,N',N'-tetramethylethylenediamine (640ul, 4.27mmol). A solution of 2,4-bis(trifluoromethyl)benzoic acid (0.50g, 1.94mmol) in dry THF (2ml) was added dropwise over 30 minutes and allowed to stir for a further 30 minutes at -80°C. lodomethane (483ul, 7.76mmol) was added dropwise over 5 minutes and the reaction stirred at -70°C for a further 20 minutes and allowed to warm to room temperature. Water (1ml) was added dropwise and the mixture partitioned between ethyl acetate and water. The water layer was acidified with 2M hydrochloric acid and extracted twice with ethyl acetate. The combined extracts were dried over magnesium sulphate and evaporated to afford a crude solid (416mg). NMR indicated this to be a mixture of 2-methyl-4,6-bis(trifluoromethyl)benzoic acid and recovered 2,4-bis(trifluoromethyl)benzoic acid. It was used without further purification.

### Description 4: 2-Methyl-4,6-bis(trifluoromethyl)benzoic acid - Alternative method.

Dry THF (5ml) was stirred under argon at -80°C and treated with sec-butyl lithium (4.0ml of a 1.4M solution in cyclohexane, 5.60mmol) and N,N,N',N'-tetramethylethylenediamine (640ul, 4.27mmol). A solution of 2,4-bis(trifluoromethyl)benzoic acid (0.50g, 1.94mmol) in dry THF (2ml) was now added dropwise over 30 minutes and allowed to stir for a further 30 minutes at -80°C. lodomethane (483ul, 7.76mmol) was now added dropwise over 5 minutes and the reaction stirred at -70°C for a further 20 minutes and allowed to warm to room temperature. Water (1ml) was added dropwise and the mixture partitioned between ethyl acetate and water. The water layer was acidified with 2M hydrochloric acid and extracted twice with ethyl acetate. The combined extracts were dried over magnesium sulphate and evaporated to afford a crude solid (420mg). NMR indicated this to be a mixture of 2-methyl-4,6-bis(trifluoromethyl)benzoic acid (ca.80%), ¹H NMR (CDCl₃) δ: 2.54 (3H, s), 7.73 (1H, s), 7.81 (1H, s), and recovered 2,4-bis(trifluoromethyl)benzoic acid (ca.20%).

### Description: 5: 2-Methyl-4,6-bis(trifluoromethyl)benzoyl chloride

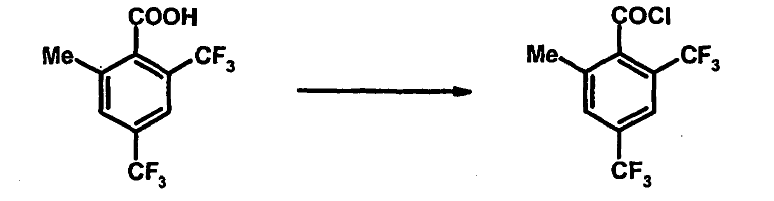

A solution of 2-methyl-4,6-bis(trifluoromethyl)benzoic acid D4 alternative method (400mg, approximately 1.47mmol) in DCM (5ml), containing DMF (1drop), was treated with oxalyl chloride (166ul, 1.91 mmol) and stirred under argon for 1 hour. The solvent was carefully removed under reduced pressure and the residue re-evaporated from further DCM. The mixture of acid chlorides was then treated with methanol (3ml) and kept at room temperature for 2 hours after which time the solvent was again carefully removed under reduced pressure. NMR data indicated this to be a mixture of 2-methyl-4,6-bis(trifluoromethyl)benzoyl chloride and methyl 2,4-bis(trifluoromethyl)benzoate. The mixture was used without further purification.

### Example 1: (±)-4-Chloro-N-[2-(dimethylamino)-2-methyl-1-phenylpropyl]-2-methyl-6-(methylthio)benzamide

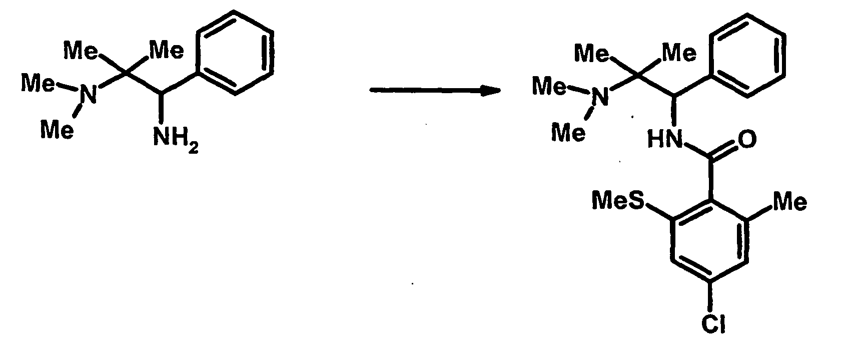

To PS-EDC (0.068g; 0.1 mmol; 1.42mmol/g) was added a solution of HOAt (0.01 mmol in 0.8ml (THF:DCM,1:1)) followed by the addition of 4-chloro-2-methyl-6-(methylthio)benzoic acid (obtainable as described in F.P. Doyle, J.H.C. Nayler, H.R.J. Waddington, J.C. Hanson and G.R. Thomas. J.Chem.Soc. 1963, 497) (0.01g; 0.05mmol) in 1:3 NMP:THF(0.25ml) and then (±)-(2-amino-1,1-dimethyl-2-phenylethyl)dimethylamine D2 (0.01g 0.05mmol) in DCM (0.25ml). The reaction was allowed to mix for 60h. Following this PS-isocyanate (0.068g, 0.1mmol, 1.5mmol/g) and PS-CO3 (0.068g, 0.1mmol, 1.5mmol/g) were added and allowed to mix for another 24h. The reaction mixture was filtered and passed through an SCX block (500mg) (pre- wetted with DCM). The content of the Robbins block was washed with more solvent (DCM:THF, 1:1) and allowed to pass through the SCX which was then washed with DCM (2ml x2) and methanol (2ml x 2). The SCX was then eluted with 0.5M ammonia in methanol and the product containing eluent evaporated to afford the title product (13.2mg; 68%). Mass Spectrum (Electrospray LC/MS): Found 391 (MH⁺). C₂₁H₂₇³⁵ClN₂OS requires 390. Ret. Time 2.47min*.

### Example 2: (±)-N-[2-(Dimethylamino)-2-methyl-1-phenylpropyl]-2-methyl-6-(methyloxy)benzamide

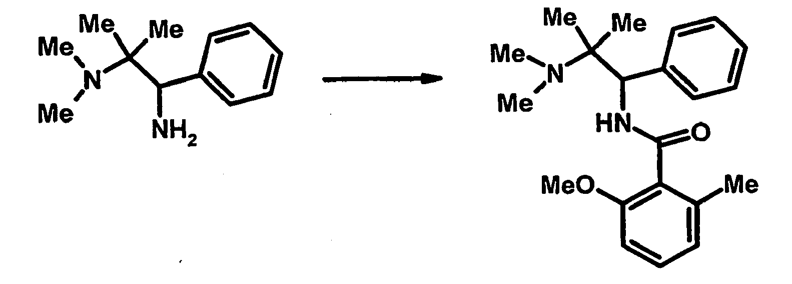

A mixture of (±)-(2-amino-1,1-dimethyl-2-phenylethyl)dimethylamine D2 (0.070g; 0.36mmol), 2-methoxy-6-methylbenzoic acid (0.091g; 0.55mmol), HOBt (0.084g; 0.55mmol), and PS-DCC (0.600g of resin loading 1.3mmol/g) in DCM (6ml) were shaken for 22 hours, filtered and the resin washed with DCM (2 x 4ml). Combined DCM organics were washed with saturated sodium hydrogen carbonate (20ml) and applied to a 2g SCX column. The SCX column was washed with DCM (2 volumes), 50%DCM in methanol (1 volume) and methanol (2 volumes) and the product eluted with 1 M ammonia in methanol (2 volumes). Evaporation afforded the title compound as a colourless gum (0.110g; 88%). ¹H NMR (CDCl₃) δ: 0.89 (3H, s), 0.94 (3H, s), 2.26 (6H, s), 2.31 (3H, s), 3.82 (3H, s), 4.90 (1H, d, J = 3.6Hz), 6.75 - 6.78 (1H, d, J = 8.4Hz), 6.79 - 6.82 (1 H, d, J = 7.6Hz), 7.15 - 7.40 (7H, m). Mass spectrum (Electrospray LC/MS), ES⁺: Found 341 (MH⁺). C₂₁H₂₈N₂O₂ requires 340. Ret. time 1.73min. The title compound was converted to its hydrochloride salt by a conventional method.

### Example 3: N-[2-(Dimethylamino)-2-methyl-1-phenylpropyl]-2,6-dimethyl benzamide chiral

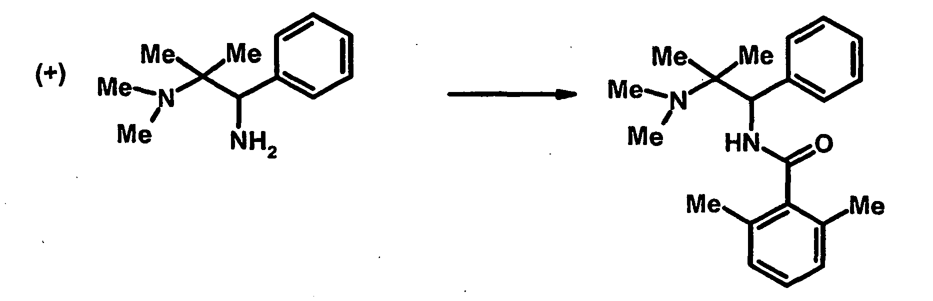

To a solution of (+)-(2-amino-1,1-dimethyl-2-phenylethyl)dimethylamine D3 (0.156g, 0.813mmol) and triethylamine (0.226ml, 1.62mmol) in DCM (8ml) was added a solution of 2,6-dimethylbenzoyl chloride (0.164g, 0.975mmol) in DCM (2ml) dropwise with cold water bath cooling. The mixture was stirred for 2h. Then saturated aqueous sodium hydrogen carbonate (30ml) was added and stirring continued for 0.25h. The organic layer was passed through a phase separation cartridge and evaporated *in-vacuo.* The residue was dissolved in DCM (5ml) and applied to a 5g SCX column. The column was washed with DCM (3 column volumes), 50% methanol in DCM (1 column volume) and methanol (3 column volumes). Elution with 1 M ammonia in methanol (2 column volumes) and evaporation of the solvent afforded the title compound as a colourless solid (0.25g, 95%). ¹H NMR (CDCl₃) δ: 0.90 (3H, s), 0.92 (3H, s), 2.25.(6H, s), 2.35 (6H, s), 4.97 (1H, d, J = 4Hz), 6.94 (1H, br s), 7.02 - 7.04 (2H, m), 7.17 (1H, m), 7.24 - 7.38 (5H, m). Mass Spectrum (Electrospray LC/MS): Found 325 (MH⁺). C₂₁H₂₈N₂O requires 324. Ret. time 1.84min. The title compound was converted to its hydrochloride salt (280mg).

### Example 4: 4-Chloro-N-[2-(dimethylamino)-2-methyl-1-phenylpropyl]-2,6-dimethyl benzamide chiral

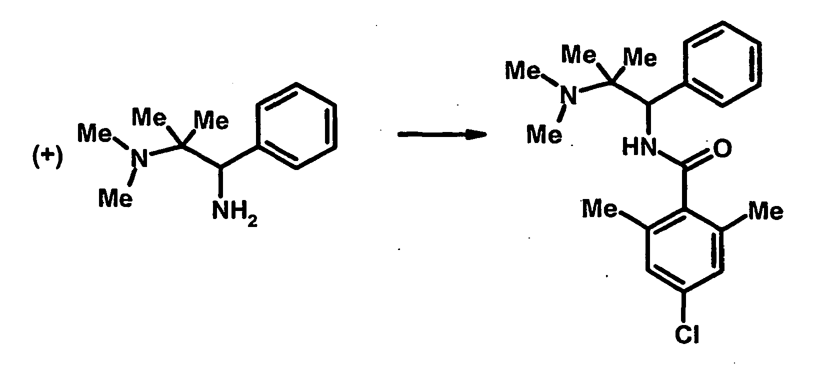

A solution of (+)-(2-amino-1,1-dimethyl-2-phenylethyl)dimethylamine D3 (0.055g, 0.29mmol) and 4-chloro-2,6-dimethylbenzoic acid (0.053g, 0.29mmol) in DCM (2 ml) was treated with HOBt (0.021g, 0.12mmol) and EDC (0.061g, 0.32mmol) and kept overnight at room temperature. The solution was washed with saturated aqueous sodium hydrogen carbonate, dried and evaporated to give a crude product which was chromatographed on silica gel (5g). Elution with 20 to 100% ethyl acetate in pentane gave the desired product as a colourless solid (0.075g, 72%). ¹H NMR (CDCl₃) δ: 0.90 (3H, s), 0.93 (3H, s), 2.26 (6H, s), 2.32 (6H, s), 4.95 (1 H, d, J = 4.0 Hz), 6.96 (1H, br s), 7.03 (2H, s), 7.26 - 7.35 (5H, overlapping m). Mass spectrum (Electrospray LC/MS): Found 359 (MH⁺). C₂₁H₂₇³⁵ClN₂O requires 358. Ret. time 2.08 min.

### Example 5: N-[2-(Dimethylamino)-2-methyl-1-phenylpropyl]-2-(methyloxy)-4,6-bis(trifluoromethyl)benzamide chiral

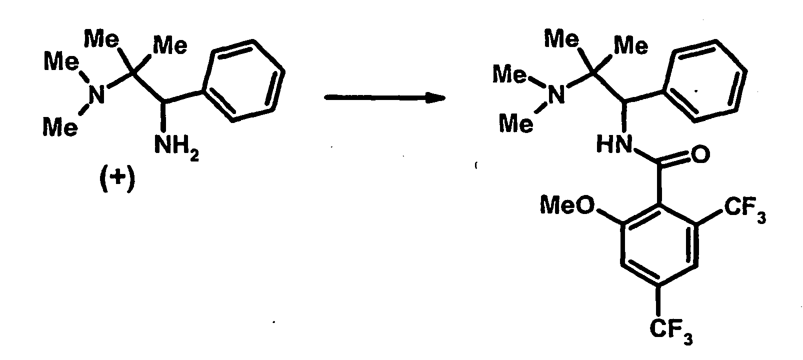

To a stirred solution of (+)-(2-amino-1,1-dimethyl-2-phenylethyl)dimethylamine D3 (0.860g, 4.48mmol) in DCM (50ml) under argon was added triethylamine (0.93ml, 6.72mmol). The solution was then chilled in ice and a solution of 2,4-ditrifluoromethyl-6-methoxy-benzoyl chloride (2.06g; 6.72mmol) in DCM (10ml) added dropwise. Cooling was removed and the reaction mixture was allowed to reach room temperature and stirred for 20h. Saturated aqueous sodium bicarbonate was added and the mixture vigorously stirred. The organic phase was separated, dried and evaporated under reduced pressure. The residue was chromatographed on silica gel, eluting with an ethyl acetate-hexane gradient (0 to 100%) to afford the title product as a white amorphous solid (1.77g; 86%). ¹H NMR (CDCl₃) δ: 0.89 (3H, s), 0.93 (3H, s), 2.24 (6H, s), 3.95 (3H, s), 4.84 (1H, d, J = 2Hz), 7.24 - 7.39 (7H, m), 7.54 (1 H, s). Mass spectrum (Electrospray LC/MS): Found 463 (MH⁺). C₂₂H₂₄F₆N₂O₂ requires 462. Ret. time 2.11min.

The product was converted to its hydrochloride salt by dissolving in methanol (50ml) and 1M HCl/diethylether was added (7.66ml, 2 equivalents). The mixture was evaporated and dried under reduced pressure to give a white amorphous solid (1.71g).

### Example 5: N-[2-(Dimethylamino)-2-methyl-1-phenylpropyl]-2-(methyloxy)-4,6-bis(trifluoromethyl)benzamide hydrochloride salt chiral- alternative method

### Step 1: (±)-(2-Amino-1,1-dimethyl-2-phenylethyl)dimethylamine (D2)

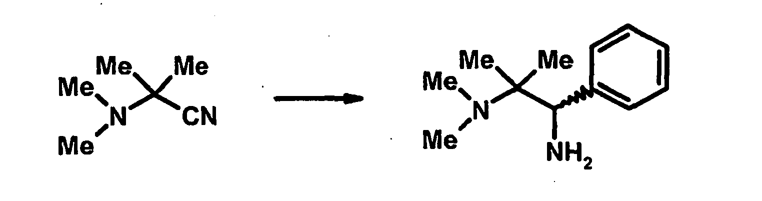

To a solution of 2-(dimethylamino)-2-methylpropanenitrile D1 (50g; 446mmol) in dry THF (1L) under nitrogen, cooled at -78°C was added dropwise a solution of phenyl lithium in dibutyl ether (469mL of a 1.9M solution; 892mmol). After 2h the reaction was allowed to reach room temperature and stirred overnight. The mixture was quenched at 0°C with a saturated solution of NaHCO₃ (ca.1L) and water (ca.600mL). The phases were separated and the aqueous back extracted with ether (1L x2). The collected organics were washed with brine 0.5L and water 0.5L,dried over Na₂SO₄ and evaporated in vacuo to get a yellow oil (94g) that was dissolved in methanol (1L) at 0°C and treated with sodium borohydride (33.74g; 892mmol). After stirring overnight at room temperature the mixture was quenched with water (0.5L). Methanol was evaporated in vacuo and the aqueous phase diluted with water (300mL) and extracted with DCM (800mL x3). The collected organics were dried over Na₂SO₄ and evaporated in vacuo to get the title product as a yellow oil (85.6g) used in step 2 without further purification.

### Step 2: (2-Amino-1,1-dimethyl-2-phenylethyl)dimethylamine R(-)α methoxy phenyl acetic acid salt (D4)

(±)-(2-amino-1,1-dimethyl-2-phenylethyl)dimethylamine D2 from step 1 (84.5g; 440mmol) was dissolved in IPA, (1.27L, 15 volumes, relative volumes being referred to the quantity of (2-amino-1,1-dimethyl-2-phenylethyl)dimethylamine). To this stirred solution heated at 50°C was added a solution of R(-)α methoxy phenyl acetic acid (440mmol) in isopropanol (422mL, 5 volumes, relative volumes being referred to the quantity of (2-amino-1,1-dimethyl-2-phenylethyl)dimethylamine). After 2h the mixture was cooled to room temperature, and the solid recovered by filtration. This solid (68g) was suspended in isopropanol (1.7L, 25 volumes, relative volumes being referred to the quantity of solid obtained in the filtration step) and heated at 60°C for 2h. After having cooled the mixture to room temperature the solid (62g) was recovered by filtration. This solid was suspended in isopropanol (1.55L, 25 volumes, relative volumes being referred to the quantity of solid obtained in the filtration step) and heated at 60°C for 2h. After having cooled the mixture to 40°C the solid was recovered by filtration, obtaining the title material (52g) as a white solid.

### Step 3: 2-(Methyloxy)-4,6-bis(trifluoromethyl)benzoyl chloride (D5)

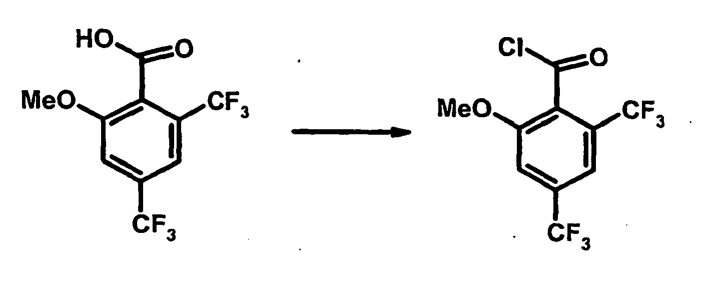

To a solution of 2-(methyloxy)-4,6-bis(trifluoromethyl)benzoic acid (23g; 79.86mmol) in dry DCM (400mL) at 0°C was added dropwise oxalyl chloride 15.32ml; 175.69mmol) followed by dry DMF (5 drops). The reaction was allowed to reach room temperature. After 3h the solvent was evaporated in vacuo to get the title product as a yellow slurry (26g) used without further purification.

### Step 4: N-[2-(Dimethylamino)-2-methyl-1-phenylpropyl]-2-(methyloxy)-4,6-bis(trifluoromethyl)benzamide chiral (E5)

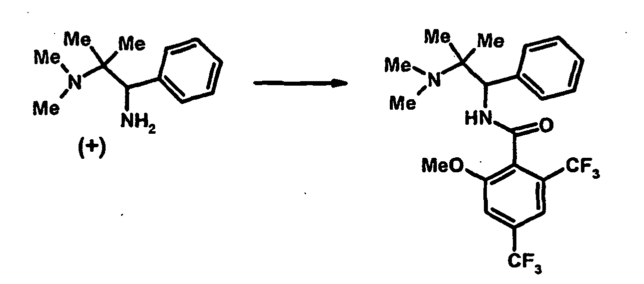

(2-Amino-1,1-dimethyl-2-phenylethyl)dimethylamine R(-)α methoxy phenyl acetic acid salt D4 from step 2 (26g; 72.62mmol) was suspended in DCM at 0°C and treated with 1M NaOH (108mL) and stirred at room temperature for 20 minutes. Water (250mL) was added to the mixture. The phases were separated, and the aqueous one was extracted with DCM (2x300mL). The collected organics were dried over Na₂SO₄ and evaporated in vacuo to get a colourless oil (11.9g) that was diluted with dry DCM (200mL) under nitrogen and cooled at 0°C. To this solution were added triethylamine (30.31 mL; 217.86mmol) and 2-(methyloxy)-4,6-bis(trifluoromethyl)benzoyl chloride D5 from step 3 (26g; 72.62mmol) dissolved in dry DCM (200mL). The reaction was left stirring at room temperature overnight and then quenched with a saturated solution of NaHCO₃ (500mL). The phases were separated and the organic washed with water, dried over Na₂SO₄ and evaporated in vacuo to get crude material that was purified by chromatography eluting with DCM/methanol from 98/2 to 95/5. Evaporation of the solvent afforded the title material (20g) as a white foam.

### Step 5: N-[2-(Dimethylamino)-2-methyl-1-phenylpropyl]-2-(methyloxy)-4,6-bis(trifluoromethyl)benzamide hydrochloride salt chiral (E5 hydrochloride salt)

*N*-[2-(Dimethylamino)-2-methyl-1-phenylpropyl]-2-(methyloxy)-4,6-bis(trifluoromethyl) benzamide E5 from step 4 (18.6g; 40.26mmol) was dissolved in dry ethyl ether (186mL, 10 volumes, relative volumes being referred to the quantity of *N*-[2-(Dimethylamino)-2-methyl-1-phenylpropyl]-2-(methyloxy)-4,6-bis(trifluoromethyl) benzamide), cooled to 0°C and treated with 1M solution of HCl in ethyl ether (42.3mL; 44.41mmol). After 0.5h the solid was collected by filtration, washed with ether and pentane, dried at 40°C overnight to get the title material (19g) as a white solid.

The compounds in the table below were prepared using similar methods to those described for the Examples above. The coupling reagents used varied: A = Acid chloride (using method similar to that of Example 3); E = EDC (using method similar to that in Example 4); P = Polymer-supported DCC (using method similar to that of Example 2); PE = Polymer-supported EDC (using method similar to that of Example 1). Work-up and purification was carried out using appropriate methods similar to those described in the examples above.

Benzoic acid starting materials were obtained commercially except for 2,6-dichloro-3-trifluoromethylbenzoic acid used for the examples indicated with a #, which was obtained by the method described in DE1924766. 4-chloro-2-methyl-6-(methylthio)benzoic acid is obtainable as described in F.P. Doyle, J.H.C. Nayler, H.R.J. Waddington, J.C. Hanson and G.R. Thomas. J.Chem.Soc. 1963, 497.

For the example compound names, those denoted by (±)- are derived from the racemic amine D2 and those without are from the chiral amine D3. LCMS retention times were generally measured using analytical LC/MS chromatography conditions method A. Compounds annoted with * are compounds prepared using array format described in Example 1 and they were analysed using analytical LC/MS chromatography conditions method B.

**Table 1**

| **Ex** | **Structure** | **Method** | **Mass spectrum (Electrospray LC/MS), API⁺ Ret.time (min)** | **Name** |
|---|---|---|---|---|
| 6 | 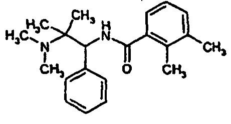 | P | Found 325 (MH⁺) C₂₁H₂₈N₂O requires 324; 1.79. | (±)-*N*-[2-(Dimethylamino)-2-methyl-1-phenylpropyl]-2,3-dimethylbenzamide |
| 7 | 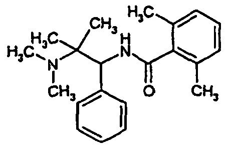 | P | Found 325 (MH⁺) C₂₁H₂₈N₂O requires 324; 1.68. | (±)-*N*-[2-(Dimethylamino)-2-methyl-1-phenylpropyl]-2,6-dimethylbenzamide |
| 8 | 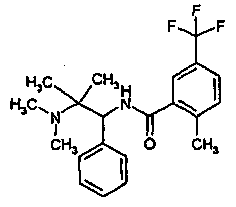 | P | Found 379 (MH⁺) C₂₁H₂₅F₃N₂O requires 378; 1.99. | (±)-*N*-[2-(Dimethylamino)-2-methyl-1-phenylpropyl]-2-methyl-5-(trifluoromethyl)benzamide |
| 9 | 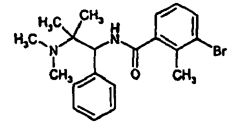 | P | Found 389 (MH⁺) C₂₀H₂₅⁷⁹BrN₂O requires 388; 2.00 | 3-bromo-*N*-[2-(dimethylamino)-2-methyl-1-phenylpropyl]-2-methylbenzamide chiral |
| 10 | 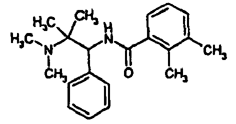 | P | Found 325 (MH⁺) C₂₁H₂₈N₂O requires 324; 1.91 | *N*-[2-(dimethylamino)-2-methyl-1-phenylpropyl]-2,3-dimethylbenzamide chiral |
| 11 | 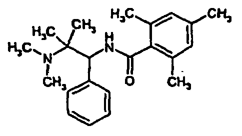 | P | Found 339 (MH⁺) C₂₂H₃₀N₂O requires 338; 1.98 | *N*-[2-(dimethylamino)-2-methyl-1-phenylpropyl]-2,4,6-trimethylbenzamide chiral |
| 12 | 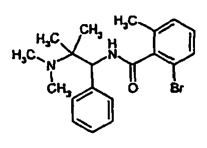 | P | Found 389 (MH⁺) C₂₀H₂₅⁷⁹BrN₂O requires 388; 1.80 | 2-bromo-*N*-[2-(dimethylamino)-2-methyl-1-phenylpropyl]-6-methylbenzamide chiral |
| 13 | 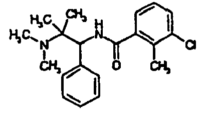 | P | Found 345 (MH⁺) C₂₀H₂₅³⁵ClN₂O requires 344; 1.97 | 3-chloro-*N*-[2-(dimethylamino)-2-methyl-1-phenylpropyl]-2-methylbenzamide chiral |
| 14 | 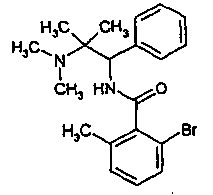 | P | Found 389 (MH⁺) C₂₀H₂₅⁷⁹BrN₂O requires 388; 1.90 | (±)-2-bromo-*N*-[2-(dimethylamino)-2-methyl-1-phenylpropyl]-6-methylbenzamide |
| 15 | 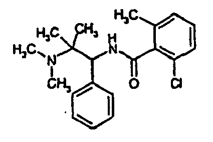 | P | Found 345 (MH⁺) C₂₀H₂₅³⁵ClN₂O requires 344; 1.78 | 2-chloro-*N*-[2-(dimethylamino)-2-methyl-1-phenylpropyl]-6-methylbenzamide chiral |
| 16 | 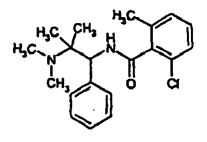 | P | Found 345 (MH⁺) C₂₀H₂₅³⁵ClN₂O requires 344; 1.78 | (±)-2-chloro-*N*-[2-(dimethylamino)-2-methyl-1-phenylpropyl]-6-methylbenzamide |
| 17 | 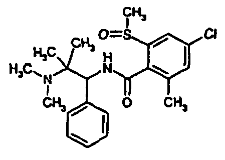 | P | Found 407 (MH⁺) C₂₁H₂₇³⁵ClN₂O₂S requires 406; 1.91 and 1.95 | 4-chloro-*N*-[2-(dimethylamino)-2-methyl-1-phenylpropyl]-2-methyl-6-(methylsulfinyl)benzamide chiral |
| 18 | 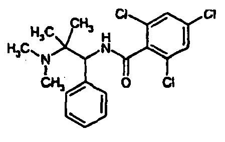 | P | Found 399 (MH⁺) C₁₉H₂₁³⁵Cl₃N₂O requires 398; 1.99 | 2,4,6-trichloro-*N*-[2-(dimethylamino)-2-methyl-1-phenylpropyl]benzamide chiral |
| 19 | 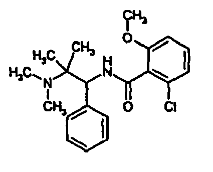 | P | Found 361 (MH⁺) C₂₀H₂₅³⁵ClN₂O₂ requires 360; 1.76 | 2-chloro-*N*-[2-(dimethylamino)-2-methyl-1-phenylpropyl]-6-(methyloxy)benzamide chiral |
| 20 | 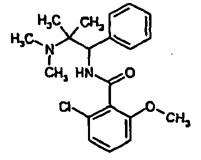 | P | Found 361 (MH⁺) C₂₀H₂₅³⁵ClN₂O₂ requires 360; 1.80 | (±)-2-Chloro-*N*-[2-(dimethylamino)-2-methyl-1-phenylpropyl]-6-(methyloxy)benzamide |
| 21 | 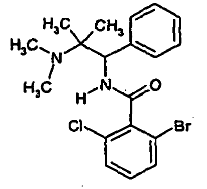 | P | Found 409 (MH⁺) C₁₉H₂₂⁷⁹Br³⁵ClN₂ O requires 408; 1.85 | (±)-2-Bromo-6-chloro-*N*-[2-(dimethylamino)-2-methyl-1-phenylpropyl]benzamide |
| 22 | 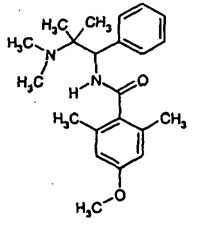 | P | Found 355 (MH⁺) C₂₂H₃₀N₂O₂ requires 354; 1.90 | (±)-*N*-[2-(Dimethylamino)-2-methyl-1-phenylpropyl]-2,6-dimethyl-4-(methyloxy)benzamide |
| 23 | 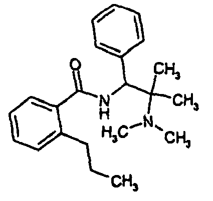 | PE | Found 339 (MH⁺) C₂₂H₃₀N₂O requires 338; 2.42* | *N*-[2-(dimethylamino)-2-methyl-1-phenylpropyl]-2-propylbenzamide chiral |
| 24 | 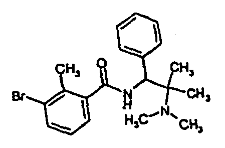 | PE | Found 389 (MH⁺) C₂₀H₂₅⁷⁹BrN₂O requires 388; 2.64* | (±)-3-bromo-*N*-[2-(dimethylamino)-2-methyl-1-phenylpropyl]-2-methylbenzamide |
| 25 | 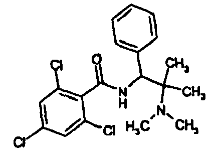 | PE | Found 399 (MH⁺) C₁₉H₂₁³⁵Cl₃N₂O requires 398; 2.91* | (±)-2,4,6-trichloro-*N*-[2-(dimethylamino)-2-methyl-1-phenylpropyl]benzamide |
| 26 | 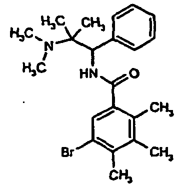 | A | Found 417 (MH⁺) C₂₂H₂₉⁷⁹BrN₂O requires 416; 2.14 | 5-bromo-*N*-[2-(dimethylamino)-2-methyl-1-phenylpropyl]-2,3,4-trimethylbenzamide chiral |
| 27 | 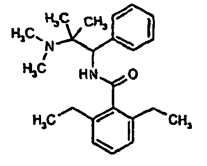 | E | Found 353 (MH⁺) C₂₃H₃₂N₂O requires 352; 2.00 | *N*-[2-(dimethylamino)-2-methyl-1-phenylpropyl]-2,6-diethylbenzamide chiral |
| 28 | 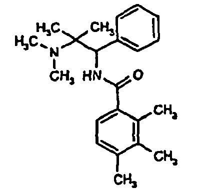 | E | Found 339 (MH⁺) C₂₂H₃₀N₂O requires 338; 1.98 | N-[2-(dimethylamino)-2-methyl-1-phenylpropyl]-2,3,4-trimethylbenzamide chiral |
| 29 | 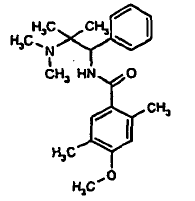 | E | Found 355 (MH⁺) C₂₂H₃₀N₂O₂ requires 354; 1.92 | *N*-[2-(dimethylamino)-2-methyl-1-phenylpropyl]-2,5-dimethyl-4-(methyloxy)benzamide chiral |
| 30 | 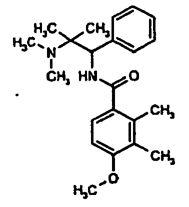 | E | Found 355 (MH⁺) C₂₂H₃₀N₂O₂ requires 354; 1.88 | *N*-[2-(dimethylamino)-2-methyl-1-phenylpropyl]-2,3-dimethyl-4-(methyloxy)benzamide chiral |
| 31 | 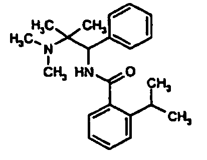 | E | Found 339 (MH⁺) C₂₂H₃₀N₂O requires 338; 1.94 | *N*-[2-(dimethylamino)-2-methyl-1-phenylpropyl]-2-(1-methylethyl)benzamide chiral |
| 32 | 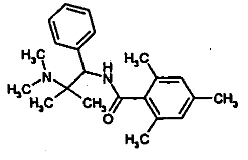 | P | Found 339 (MH⁺) C₂₂H₃₀N₂O requires 338; 1.95 | (±)-*N*-[2-(dimethylamino)-2-methyl-1-phenylpropyl]-2,4,6-trimethylbenzamide |
| 33 | 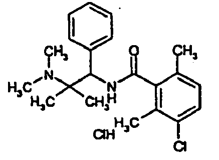 | P | Found 359 (MH⁺) C₂₁H₂₇³⁵ClN₂O requires 358; 2.03 | 3-chloro-*N*-[2-(dimethylamino)-2-methyl-1-phenylpropyl]-2,6-dimethylbenzamide chiral |
| 34 | 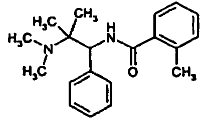 | A | Found 311 (MH⁺) C₂₀H₂₆N₂O requires 310; 1.81 | (±)-*N*-[2-(dimethylamino)-2-methyl-1-phenylpropyl]-2-methylbenzamide |
| 35 | 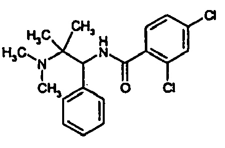 | A | Found 365 (MH⁺) C₁₉H₂₂³⁵Cl₂N₂O requires 364; 1.90 | (±)-2,4-dichloro-*N*-[2-(dimethylamino)-2-methyl-1-phenylpropyl]benzamide |
| 36 | 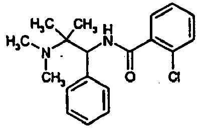 | A | Found 331 (MH⁺) C₁₉H₂₃³⁵ClN₂O requires 330; 1.71 | (±)-2-chloro-*N*-[2-(dimethylamino)-2-methyl-1-phenylpropyl]benzamide |
| 37 | 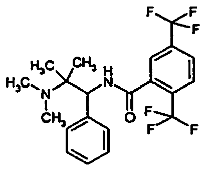 | A | Found 433 (MH⁺) C₂₁H₂₂F₆N₂O requires 432; 2.11 | (±)-*N*-[2-(dimethylamino)-2-methyl-1-phenylpropyl]-2,5-bis(trifluoromethyl)benzamid e |
| 38 | 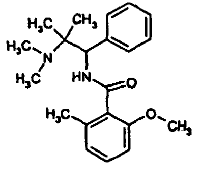 | P | Found 341 (MH⁺) C₂₁H₂₈N₂O₂ requires 340; 1.73. | *N*-[2-(dimethylamino)-2-methyl-1-phenylpropyl]-2-methyl-6-(methyloxy)benzamide chiral |
| 39 | 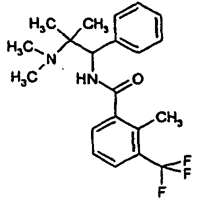 | P | Found 379 (MH⁺) C₂₁H₂₅F₃N₂O requires 378; 1.99 | (±)-*N*-[2-(dimethylamino)-2-methyl-1-phenylpropyl]-2-methyl-3-(trifluoromethyl)benzamide |
| 40 | 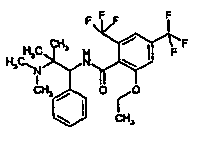 | E | Found 477 (MH⁺) C₂₃H₂₆F₆N₂O₂ requires 476; 2.14. | *N*-[2-(dimethylamino)-2-methyl-1-phenylpropyl]-2-(ethyloxy)-4,6-bis(trifluoromethyl)benzamid e chiral |
| 41 | 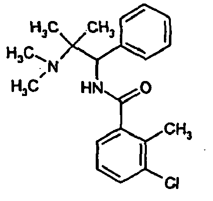 | P | Found 345 (MH⁺) C₂₀H₂₅³⁵ClN₂O requires 344; 1.9. | (±)-3-chloro-*N*-[2-(dimethylamino)-2-methyl-1-phenylpropyl]-2methylbenzamide |
| 42 | 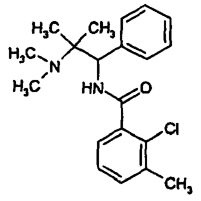 | P | Found 345 (MH⁺) C₂₀H₂₅³⁵ClN₂O requires 344; 1.79 | (±)-2-chloro-*N*-[2-(dimethylamino)-2-methyl-1-phenylpropyl]-3-methylbenzamide |
| 43 | 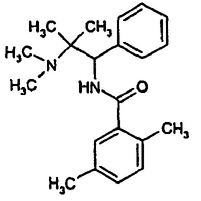 | P | Found 325 (MH⁺) C₂₁H₂₈N₂O requires 324; 1.8. | (±)-*N*-[2-(dimethylamino)-2-methyl-1-phenylpropyl]-2,5-dimethylbenzamide |
| 44 | 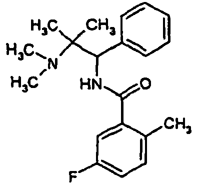 | P | Found 329 (MH⁺) C₂₀H₂₅FN₂O requires 328; 1.69. | (±)-*N*-[2-(dimethylamino)-2-methyl-1-phenylpropyl]-5-fluoro-2-methylbenzamide |
| 45 | 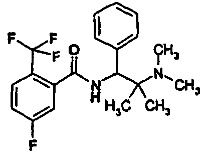 | A | Found 383 (MH⁺) C₂₀H₂₂F₄N₂O requires 382; 1.83 | *N*-[2-(dimethylamino)-2-methyl-1-phenylpropyl]-5-fluoro-2-(trifluoromethyl)benzamide chiral |
| 46 | 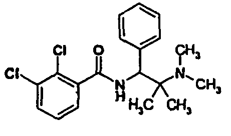 | A | Found 365 (MH⁺) C₁₉H₂₂³⁵Cl₂N₂O requires 364; 1.89 | 2,3-dichloro-*N*-[2-(dimethylamino)-2-methyl-1-phenylpropyl]benzamide chiral |
| 47 | 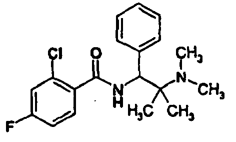 | A | Found 349 (MH⁺) C₁₉H₂₂³⁵ClFN₂O requires 348; 1.75 | 2-chloro-*N*-[2-(dimethylamino)-2-methyl-1-phenylpropyl]-4-fluorobenzamide chiral |
| 48 | 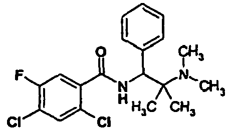 | A | Found 383 (MH⁺) C₁₉H₂₁³⁵Cl₂FN₂O requires 382; 1.93 | 2,4-dichloro-*N*-[2-(dimethylamino)-2-methyl-1-phenylpropyl]-5-fluorobenzamide chiral |
| 49 | 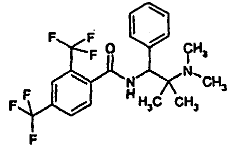 | A | Found 433 (MH⁺) C₂₁H₂₂F₆N₂O Requires 432; 2.08 | *N*-[2-(dimethylamino)-2-methyl-1-phenylpropyl]-2,4-bis(trifluoromethyl)benzamid e chiral |
| 50 | 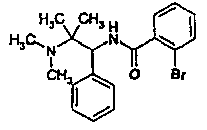 | A | Found 375(MH⁺) C₁₉H₂₃⁷⁹BrN₂O requires 374; 1.66 | (±)-2-bromo-*N*-[2-(dimethylamino)-2-methyl-1-phenylpropyl]benzamide |
| 51 | 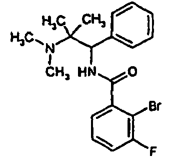 | P | Found 393(MH⁺) C₁₉H₂₂⁷⁹BrFN₂O requires 392; 1.89. | (±)-2-bromo-*N*-[2-(dimethylamino)-2-methyl-1-phenylpropyl]-3-fluorobenzamide |
| 52 | 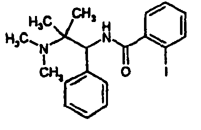 | A | Found 423(MH⁺) C₁₉H₂₃IN₂O requires 422; 1.73. | (±)-*N*-[2-(dimethylamino)-2-methyl-1-phenylpropyl]-2-iodobenzamide |
| 53 | 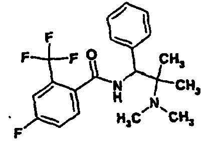 | PE | Found 383(MH⁺) C₂₀H₂₂F₄N₂O requires 382; 2.34.* | (±)-*N*-[2-(dimethylamino)-2-methyl-1-phenylpropy]-4-fluoro-2-(trifluoromethyl)benzamide |
| 54 | 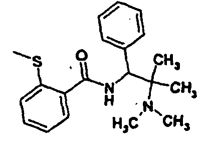 | PE | Found 343(MH⁺) C₂₀H₂₆N₂OS requires 342; 2.49.* | (±)-*N*-[2-(dimethylamino)-2-methyl-1-phenylpropyl]-2-(methylthio)benzamide |
| 55 | 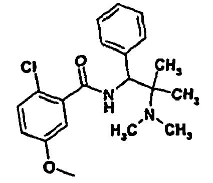 | PE | Found 361 (MH⁺) C₂₀H₂₅³⁵ClN₂O₂ requires 360; 2.45.* | (±)-2-chloro-*N*-[2(dimethylamino)-2-methyl-1-phenylpropyl]-5-(methyloxy)benzamide |
| 56 | 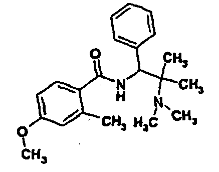 | PE | Found 341 (MH⁺) C₂₁H₂₈N₂O₂ requires 340; 2.81.* | (±)-*N*-[2-(dimethylamino)-2-methyl-1-phenylpropyl]-2-methyl-4-(methyloxy)benzamide |
| 57 | 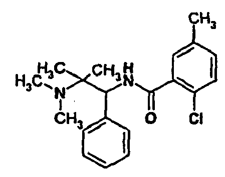 | P | Found 345 (MH⁺) C₂₀H₂₅³⁵ClN₂O requires 344; 1.83. | (±)-2-chloro-*N*-[2-(dimethylamino)-2-methyl-1-phenylpropyl]-5-methylbenzamide |
| 58 | 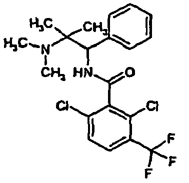 | P# | Found 433 (MH⁺) C₂₀H₂₁³⁵Cl₂F₃N₂O requires 432; 2.04 | 2,6-dichloro-*N*-[2-(dimethylamino)-2-methyl-1-phenylpropyl]-3-(trifluoromethyl)benzamide chiral |
| 59 | 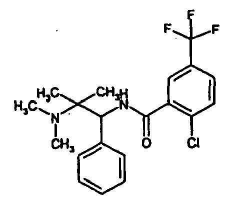 | P | Found 399 (MH⁺) C₂₀H₂₂³⁵ClF₃N₂O requires 398. 1.95 | (±)-2-chloro-*N*-[2-(dimethylamino)-2-methyl-1-phenylpropyl]-5-(trifluoromethyl)benzamide |
| 60 | 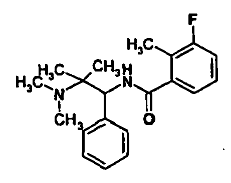 | P | Found 329 (MH⁺) C₂₀H₂₅FN₂O requires 328; 1.88. | (±)-*N*-[2-(dimethylamino)-2-methyl-1-phenylpropyl]-3-fluoro-2-methylbenzamide |
| 61 | 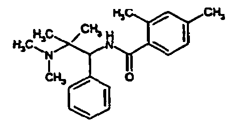 | P | Found 325 (MH⁺) C₂₁H₂₈N₂O requires 324; 1.90. | (±)-*N*-[2-(dimethylamino)-2-methyl-1-phenylpropyl]-2,4-dimethylbenzamide |
| 62 | 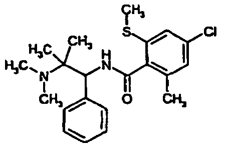 | P | Found 391 (MH⁺) C₂₁H₂₇³⁵ClN₂OS requires 390; 2.10 | 4-chloro-*N*-[2-(dimethylamino)-2-methyl-1-phenylpropyl]-2-methyl-6-(methylthio)benzamide chiral |
| 63 | 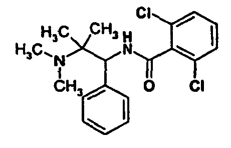 | P | Found 365 (MH⁺) C₁₉H₂₂³⁵Cl₂N₂o requires 364; 1.70. | (±)-2,6-dichloro-*N*-[2-(dimethylamino)-2-methyl-1-phenylpropyl]benzamide |
| 64 | 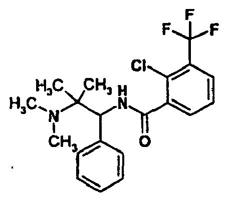 | P | Found 399 (MH⁺) C₂₀H₂₂³⁵ClF₃N₂O requires 398; 2.00. | (±)-2-chloro-*N*-[2-(dimethylamino)-2-methyl-1-phenylpropyl]-3-(trifluoromethyl)benzamide |

| | | | | |
|---|---|---|---|---|
| # 2,6-dichloro-3-trifluoromethylbenzoic acid (DE1924766) | | | | |

### Example 65: N-[2-(Dimethylamino)-2-methyl-1-phenylpropyl]-2-methyl-4,6-bis(trifluoromethyl)benzamide chiral

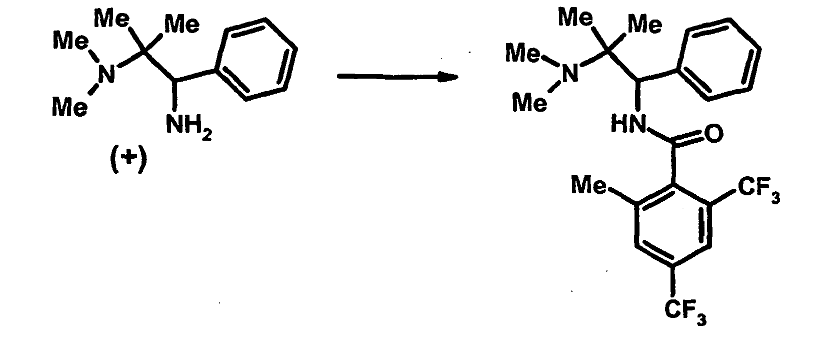

A mixture (approximately 0.50mmol) of 2-methyl-4,6-bis(trifluoromethyl)benzoyl chloride and methyl 2,4-bis(trifluoromethyl)benzoate prepared as described in D5 was dissolved in dry DCM (3ml) and treated with (+)-(2-amino-1,1-dimethyl-2-phenylethyl)dimethylamine D3 (70mg, 0.36 mmol) and triethylamine (140ul, 1.00 mmol) and left overnight at room temperature. The volatile components were removed under reduced pressure and the residue chromatographed on silica gel. Elution with 0-80% ethyl acetate in pentane gave the title product as a gum (70mg, approximately 44%). ¹H NMR (CDCl₃) δ: 0.89 (3H, s), 0.94 (3H, s), 2.24 (6H, s), 2.48 (3H, s), 4.89 (1H, d, J = 2.8 Hz), 7.18 (1H, br s), 7.27-7.37 (5H, overlapping m), 7.68 (1H, s), 7.78 (1H, s). Mass spectrum (Electrospray LC/MS): Found 447 (MH⁺) C₂₂H₂₄ F₆N₂O requires 446. Ret time 2.16 min.

The title product was converted to the hydrochloride salt (81 mg) by addition of excess 1M HCl in ether to a chloroform solution of the amine and removal of the solvent under reduced pressure.

The compounds of the Examples above could be converted to their corresponding hydrochloride salts by dissolving the parent free base in DCM or DCM / methanol mixtures and adding 1 M hydrogen chloride in ether, followed by evaporation and drying under reducd pressure. Compounds purified by Mass Directed Auto-Purification were isolated as the formate salt which could be converted to the free base via an SCX column and to the corresponding hydrochloride salt by reaction with 1M hydrogen chloride in ether as described above.

## Claims

1. A compound of formula (I) or a salt or solvate thereof: 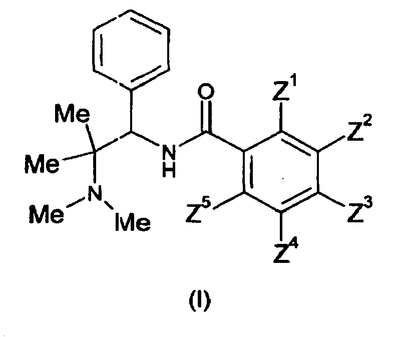 wherein
Z¹ is selected from the group consisting of C₁₋₄alkyl, C₃₋₆cycloalkyl, C₁₋₄alkylthio, haloC₁-₄alkyl, phenyl, halophenyl, C₁₋₄alkylsulfoxy, C₁₋₄alkylsulfonyl, chloro, bromo or iodo;
Z² is selected from the group consisting of hydrogen, halogen, C₁₋₄alkyl, phenyl, haloC₁-₄alkyl, haloC₁₋₄alkoxy, halophenyl, C₁₋₄alkoxyC₁₋₄alkyl and C₃₋₆cycloalkyl;
Z³ is selected from the group consisting of hydrogen, halogen, C₁₋₄alkyl, C₁₋₄alkoxy, C₁-₄alkylthio, haloC₁₋₄alkyl, haloC₁₋₄alkoxy, and C₃₋₆cycloalkyl;
Z⁴ is selected from the group consisting of hydrogen, halogen, C₁₋₄alkyl, haloC₁₋₄alkyl, C₁-₄alkoxy, C₁₋₄alkylthio, phenyl, haloC₁₋₄alkoxy, halophenyl, C₁₋₄alkoxyC₁₋₄alkyl and C₃₋₆cycloalkyl;
Z⁵ is selected from the group consisting of hydrogen, chloro, bromo, iodo, hydroxy, C₁-₄alkyl, C₁₋₄alkoxy, C₁₋₄alkylthio, phenyl, haloC₁₋₄alkoxy, halophenyl, C₁₋₄alkoxyC₁₋₄alkyl and C₃₋₆cycloalkyl.

2. A compound as claimed in claim 1 which is a compound of formula (Ia) or a salt or solvate thereof: 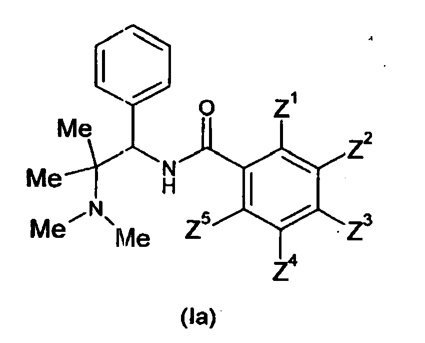 wherein
Z¹ is selected from the group consisting of C₁₋₄alkyl, C₁₋₄alkylthio, haloC₁₋₄alkyl, C₁-₄alkylsulfoxy, chloro and bromo;
Z² is selected from the group consisting of hydrogen, halogen, C₁₋₄alkyl and haloC₁₋₄alkyl;
Z³ is selected from the group consisting of hydrogen, halogen, C₁₋₄alkyl, C₁₋₄alkoxy and haloC₁₋₄alkyl;
Z⁴ is selected from the group consisting of hydrogen, halogen, C₁₋₄alkyl, C₁₋₄alkoxy and haloC₁₋₄alkyl;
Z⁵ is selected from the group consisting of hydrogen, chloro, C₁₋₄alkyl and C₁₋₄alkoxy.

3. A compound as claimed in claim 1 or claim 2 which is:
(±)-4-Chloro-*N*-[2-(dimethylamino)-2-methyl-1-phenylpropyl]-2-methyl-6-(methylthio)benzamide
(±)-*N*-[2-(Dimethylamino)-2-methyl-1-phenylpropyl]-2-methyl-6-(methyloxy)benzamide
*N*-(2-(Dimethylamino)-2-methyl-1-phenylpropyl]-2,6-dimethyl benzamide chiral
4-Chloro-*N*-[2-(dimethylamino)-2-methyl-1-phenylpropyl]-2,6-dimethyl benzamide chiral
*N*-[2-(Dimethylamino)-2-methyl-1-phenylpropyl]-2-(methyloxy)-4,6-bis(trifluoromethyl)benzamide chiral
(±)-*N*-[2-(Dimethylamino-2-methyl-1-phenylpropyl]-2,3-dimethylbenzamide
(±)-*N*-[2-(Dimethylamino)-2-methyl-1-phenylpropyl]-2,6-dimethylbenzamide
(±)-*N*-[2-(Dimethylamino)-2-methyl-1-phenylpropyl]-2-methyl-5-(trifluoromethyl)benzamide
3-bromo-*N*-[2-(dimethylamino)-2-methyl-1-phenylpropyl]-2-methylbenzamide chiral
*N*-[2-(dimethylamino)-2-methyl-1-phenylpropyl]-2,3-dimethylbenzamide chiral
*N*-[2-(dimethylamino)-2-methyl-1-phenylpropyl]-2,4,6-trimethylbenzamide chiral
2-bromo-*N*-[2-(dimethylamino)-2-methyl-1-phenylpropyl]-6-methylbenzamide chiral
3-chloro-*N*-[2-(dimethylamino)-2-methyl-1-phenylpropyl]-2-methylbenzamide chiral
(±)-2-bromo-*N*-[2-(dimethylamino)-2-methyl-1-phenylpropyl]-6-methylbenzamide
2-chloro-*N*-[2-(dimethylamino)-2-methyl-1-phenylpropyl]-6-methylbenzamide chiral
(±) -2-chloro-*N*-[2-(dimethylamino)-2-methyl-1-phenylpropyl]-6-methylbenzamide
4-chloro-*N*-[2-(dimethylamino)-2-methyl-1-phenylpropyl]-2-methyl-6-(methylsulfinyl)benzamide chiral
2,4,6-trichloro-*N*-[2-(dimethylamino)-2-methyl-1-phenylpropyl]benzamide chiral
2-chloro-*N*-[2-(dimethylamino)-2-methyl-1-phenylpropyl]-6-(methyloxy)benzamide chiral
(±)-2-Chloro-*N*-[2-(dimethylamino)-2-methyl-1-phenylpropyl]-6-(methyloxy)benzamide
(±)-2-Bromo-6-chloro-*N*-[2-(dimethylamino)-2-methyl-1-phenylpropyl]benzamide
(±)-*N*-[2-(Dimethylamino)-2-methyl-1-phenylpropyl]-2,6-dimethyl-4-(methyloxy)benzamide
*N*-[2-(dimethylamino)-2-methyl-1-phenylpropyl]-2-propylbenzamide chiral
(±)-3-bromo-*N*-[2-(dimethylamino)-2-methyl-1-phenylpropyl]-2-methylbenzamide
(±)-2,4,6-trichloro-*N*-[2-(dimethylamino)-2-methyl-1-phenylpropyl]benzamide
5-bromo-*N-*[2-(dimethylamino)-2-methyl-1-phenylpropyl]-2,3,4-trimethylbenzamide chiral
*N*-[2-(dimethylamino)-2-methyl-1-phenylpropyl]-2,6-diethylbenzamide chiral
*N*-[2-(dimethylamino)-2-methyl-1-phenylpropyl]-2,3,4-trimethylbenzamide chiral
*N*-[2-(dimethylamino)-2-methyl-1-phenylpropyl]-2,5-dimethyl-4-(methyloxy)benzamide chiral
*N*-[2-(dimethylamino)-2-methyl-1-phenylpropyl]-2,3-dimethyl-4-(methyloxy)benzamide chiral
*N*-[2-(dimethylamino)-2-methyl-1-phenylpropyl]-2-(1-methylethyl)benzamide chiral
(±)-*N*-[2-(dimethylamino)-2-methyl-1-phenylpropyl]-2,4,6-trimethylbenzamide
3-chloro-*N*-[2-(dimethylamino)-2-methyl-1-phenylpropyl]-2,6-dimethylbenzamide chiral
(±)-*N*-[2-(dimethylamino)-2-methyl-1-phenylpropyl]-2-methylbenzamide
(±)-2,4-dichloro-*N*-[2-(dimethylamino)-2-methyl-1-phenylpropyl]benzamide
(±)-2-chloro-*N*-[2-(dimethylamino)-2-methyl-1-phenylpropyl]benzamide
(±)-*N*-[2-(dimethylamino)-2-methyl-1-phenylpropyl]-2,5-bis(trifluoromethyl)benzamide
*N*-[2-(dimethylamino)-2-methyl-1-phenylpropyl]-2-methyl-6-(methyloxy)benzamide chiral
(±)-*N*-[2-(dimethylamino)-2-methyl-1-phenylpropyl]-2-methyl-3-(trifluoromethyl)benzamide
*N*-[2-(dimethylamino)-2-methyl-1-phenylpropyl]-2-(ethyloxy)-4,6-bis(trifluoromethyl)benzamide chiral
(±)-3-chloro-*N*-[2-(dimethylamino)-2-methyl-1-phenylpropyl]-2-methylbenzamide
(±)-2-chloro-*N*-[2-(dimethylamino)-2-methyl-1-phenylpropyl]-3-methylbenzamide
(±)-*N*-[2-(dimethylamino)-2-methyl-1-phenylpropyl]-2,5-dimethylbenzamide
(±)-*N*-[2-(dimethylamino)-2-methyl-1-phenylpropyl]-5-fluoro-2-methylbenzamide
*N*-[2-(dimethylamino)-2-methyl-1-phenylpropyl]-5-fluoro-2-(trifluoromethyl)benzamide chiral
2,3-dichloro-*N*-[2-(dimethylamino)-2-methyl-1-phenylpropyl]benzamide chiral
2-chloro-*N*-[2-(dimethylamino)-2-methyl-1-phenylpropyl]-4-fluorobenzamide chiral
2,4-dichloro-*N*-[2-(dimethylamino)-2-methyl-1-phenylpropyl]-5-fluorobenzamide chiral
N-[2-(dimethylamino)-2-methyl-1-phenylpropyl]-2,4-bis(trifluoromethyl)benzamide chiral
(±)-2-bromo-*N*-[2-(dimethylamino)-2-methyl-1-phenylpropyl]benzamide
(±)-2-bromo-*N*-[2-(dimethylamino)-2-methyl-1-phenylpropyl]-3-fluorobenzamide
(±)-*N-*[2-(dimethylamino)-2-methyl-1-phenylpropyl]-2-iodobenzamide
(±)-*N*-[2-(dimethylamino)-2-methyl-1-phenylpropyl]-4-fluoro-2-(trifluoromethyl)benzamide
(±)-*N*-[2-(dimethylamino)-2-methyl-1-phenylpropyl]-2-(methylthio)benzamide
(±)-2-chloro-*N*-[2-(dimethylamino)-2-methyl-1-phenylpropyl]-5-(methyloxy)benzamide
(±)-*N*-[2-(dimethylamino)-2-methyl-1-phenylpropyl]-2-methyl-4-(methyloxy)benzamide
(±)-2-chloro-*N*-[2-(dimethylamino)-2-methyl-1-phenylpropyl]-5-methylbenzamide
2,6-dichloro-*N*-[2-(dimethylamino)-2-methyl-1-phenylpropyl]-3-(trifluoromethyl)benzamide chiral
(±)-2-chloro-*N*-[2-(dimethylamino)-2-methyl-1-phenylpropyl]-5-(trifluoromethyl)benzamide
(±)-*N*-[2-(dimethylamino)-2-methyl-1-phenylpropyl]-3-fluoro-2-methylbenzamide
(±)-*N*-[2-(dimethylamino)-2-methyl-1-phenylpropyl]-2,4-dimethylbenzamide
4-chloro-*N*-[2-(dimethylamino)-2-methyl-1-phenylpropyl]-2-methyl-6-(methylthio)benzamide chiral
(±)-2,6-dichloro-*N*-[2-(dimethylamino)-2-methyl-1-phenylpropyl]benzamide
(±)-2-chloro-*N*-[2-(dimethylamino)-2-methyl-1-phenylpropyl]-3-(trifluoromethyl)benzamide
*N*-[2-(Dimethylamino)-2-methyl-1-phenylpropyl]-2-methyl-4,6-bis(trifluoromethyl)benzamide chiral
or a salt or solvate thereof.

4. A compound as claimed in any one of claims 1 to 3 for use in therapy.

5. A compound as claimed in claim 4 for use in the treatment of a disorder mediated by GlyT1.

6. A compound as claimed in claim 5, wherein the disorder is psychosis, including schizophrenia, dementia or attention deficit disorder.

7. Use of a compound as claimed in claim 1 in the preparation of a medicament for the treatment of a disorder mediated by Glut1.

8. Use as claimed in claim 7, wherein the disorder is psychosis, including schizophrenia, dementia or attention deficit disorder.

9. A pharmaceutical composition comprising a compound as claimed in claim 4, and at least one pharmaceutically acceptable carrier, diluent or excipient.

10. A pharmaceutical composition as claimed in claim 9 further comprising one or more other therapeutic agents, selected from antidepressant agents selected from 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists, anticonvulsant agents; atypical antipsychotic drugs and cognitive enhancers.

11. A method of preparing a compound as defined in formula (I) in any one of claims 1 to 3, comprising the step of reacting a compound of formula (II): 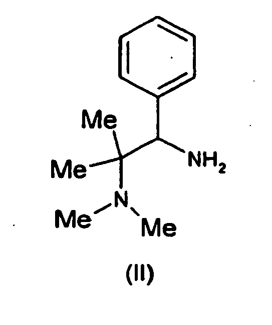 with a compound of formula (III): 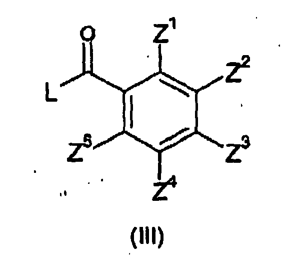 wherein Z¹, Z², Z³, Z⁴ and Z⁵ are as defined in formula (I) in any one of claims 1 to 3 and L represents a suitable leaving group;
and thereafter optionally:
• removing any protecting groups and/or
• converting a compound of formula (I) into another compound of formula (I) and/or
• forming a salt or solvate.

## Patentansprüche

1. Verbindung der Formel (I) oder ein Salz oder ein Solvat hiervon: 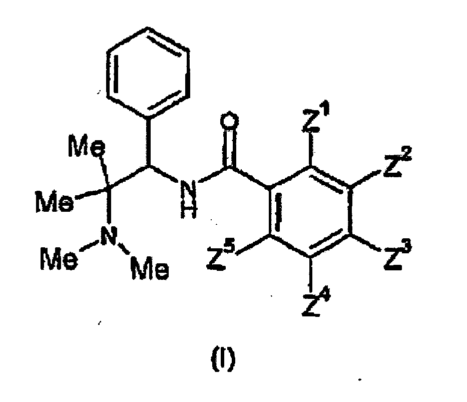 wobei
Z¹ aus C₁₋₄-Alkyl, C₃₋₆-Cycloalkyl, C₁₋₄-Alkylthio, Halogen-C₁₋₄-alkyl, Phenyl, Halogenphenyl, C₁₋₄-Alkylsulfoxy, C₁₋₄-Alkylsulfonyl, Chlor, Brom oder Iod ausgewählt ist;
Z² aus Wasserstoff, Halogen, C₁₋₄-Alkyl, Phenyl, Halogen-C₁₋₄-alkyl, Halogen-C₁₋₄-alkoxy, Halogenphenyl, C₁₋₄-Alkoxy-C₁₋₄-alkyl und C₃₋₆-Cycloalkyl ausgewählt ist;
Z³ aus Wasserstoff, Halogen, C₁₋₄-Alkyl, C₁₋₄-Alkoxy, C₁₋₄-Alkylthio, Halogen-C₁₋₄-alkyl, Halogen-C₁₋₄-alkoxy und C₃₋₆-Cycloalkyl ausgewählt ist;
Z⁴ aus Wasserstoff, Halogen, C₁₋₄-Alkyl, Halogen-C₁₋₄-alkyl, C₁₋₄-Alkoxy, C₁₋₄-Alkylthio, Phenyl, Halogen-C₁₋₄-alkoxy, Halogenphenyl, C₁₋₄-Alkoxy-C₁₋₄-alkyl und C₃₋₆-Cycloalkyl ausgewählt ist;
Z⁵ aus Wasserstoff, Chlor, Brom, Iod, Hydroxy, C₁₋₄-Alkyl, C₁₋₄-Alkoxy, C₁₋₄-Alkylthio, Phenyl, Halogen-C₁₋₄-alkoxy, Halogenphenyl, C₁₋₄-Alkoxy-C₁₋₄-alkyl und C₃₋₆-Cycloalkyl ausgewählt ist.

2. Verbindung gemäß Anspruch 1, welche eine Verbindung der Formel (Ia) oder ein Salz oder ein Solvat hiervon ist: 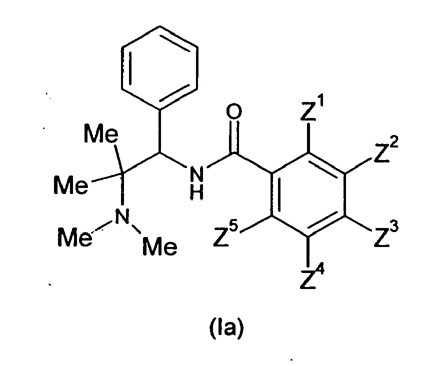 wobei
Z¹ aus C₁₋₄-Alkyl, C₁₋₄-Alkylthio, Halogen-C₁₋₄-alkyl, C₁₋₄-Alkylsulfoxy, Chlor und Brom ausgewählt ist;
Z² aus Wasserstoff, Halogen, C₁₋₄-Alkyl und Halogen-C₁₋₄-alkyl ausgewählt ist;
Z³ aus Wasserstoff, Halogen, C₁₋₄-Alkyl, C₁₋₄-Alkoxy und Halogen-C₁₋₄-alkyl ausgewählt ist;
Z⁴ aus Wasserstoff, Halogen, C₁₋₄-Alkyl, C₁₋₄-Alkoxy und Halogen-C₁₋₄-alkyl ausgewählt ist;
Z⁵ aus Wasserstoff, Chlor, C₁₋₄-Alkyl und C₁₋₄-Alkoxy ausgewählt ist.

3. Verbindung gemäß Anspruch 1 oder Anspruch 2, welche
(±)-4-Chlor-N-[2-(dimethylamino)-2-methyl-1-phenylpropyl]-2-methyl-6-(methylthio)-benzamid
(±)-N-[2-(Dimethylamino)-2-methyl-1-phenylpropyl]-2-methyl-6-(methyloxy)benzamid
N-[2-(Dimethylamino)-2-methyl-1-phenylpropyl]-2,6-dimethylbenzamid chiral
4-Chlor-N-[2-(dimethylamino)-2-methyl-1-phenylpropyl]-2,6-dimethylbenzamid chiral
N-[2-(Dimethylamino)-2-methyl-1-phenylpropyl]-2-(methyloxy)-4,6-bis(trifluormethyl)-benzamid chiral
(±)-N-[2-(Dimethylamino)-2-methyl-1-phenylpropyl]-2,3-dimethylbenzamid
(±)-N-[2-(Dimethylamino)-2-methyl-1-phenylpropyl]-2,6-dimethylbenzamid
(±)-N-[2-(Dimethylamino)-2-methyl-1-phenylpropyl]-2-methyl-5-(trifluormethyl)-benzamid
3-Brom-N-[2-(dimethylamino)-2-methyl-1-phenylpropyl]-2-methylbenzamid chiral
N-[2-(Dimethylamino)-2-methyl-1-phenylpropyl]-2,3-dimethylbenzamid chiral
N-[2-(Dimethylamino)-2-methyl-1-phenylpropyl]-2,4,6-trimethylbenzamid chiral
2-Brom-N-[2-(dimethylamino)-2-methyl-1-phenylpropyl]-6-methylbenzamid chiral
3-Chlor-N-[2-(dimethylamino)-2-methyl-1-phenylpropyl]-2-methylbenzamid chiral
(±)-2-Brom-N-[2-(dimethylamino)-2-methyl-1-phenylpropyl]-6-methylbenzamid
2-Chlor-N-[2-(dimethylamino)-2-methyl-1-phenylpropyl]-6-methylbenzamid chiral
(±)-2-Chlor-N-[2-(dimethylamino)-2-methyl-1-phenylpropyl]-6-methylbenzamid
4-Chlor-N-[2-(dimethylamino)-2-methyl-1-phenylpropyl]-2-methyl-6-(methylsulfinyl)-benzamid chiral
2,4,6-Trichlor-N-[2-(dimethylamino)-2-methyl-1-phenylpropyl]benzamid chiral
2-Chlor-N-[2-(dimethylamino)-2-methyl-1-phenylpropyl]-6-(methyloxy)benzamid chiral
(±)-2-Chlor-N-[2-(dimethylamino)-2-methyl-1-phenylpropyl]-6-(methyloxy)benzamid
(±)-2-Brom-6-chlor-N-[2-(dimethylamino)-2-methyl-1-phenylpropyl]benzamid
(±)-N-[2-(Dimethylamino)-2-methyl-1-phenylpropyl]-2,6-dimethyl-4-(methyloxy)-benzamid
N-[2-(Dimethylamino)-2-methyl-1-phenylpropyl]-2-propylbenzamid chiral
(±)-3-Brom-N-[2-(dimethylamino)-2-methyl-1-phenylpropyl]-2-methylbenzamid
(±)-2,4,6-Trichlor-N-[2-(dimethylamino)-2-methyl-1-phenylpropyl]benzamid
5-Brom-N-[2-(dimcthylamino)-2-methyl-l-phenylpropyl]-2,3,4-trimethylbenzamid chiral
N-[2-(Dimethylainino)-2-methyl-1-phenylpropyl]-2,6-diethylbenzamid chiral
N-[2-(Dimethylamino)-2-methyl-1-phenylpropyl]-2,3,4-trimethylbenzamid chiral
N-[2-(Dimethylamino)-2-methyl-1-phenylpropyl]-2,5-dimethyl-4-(methyloxy)benzamid chiral
N-[2-(Dimethylamino)-2-methyl-1-phenylpropyl]-2,3-dimethyl-4-(methyloxy)benzamid chiral
N-[2-(Dimethylamino)-2-methyl-1-phenylpropyl]-2-(1-methylethyl)benzamid chiral
(±)-N-[2-(Dimethylamino)-2-methyl-1-phenylpropyl]-2,4,6-trimethylbenzamid
3-Chlor-N-[2-(dimethylamino)-2-methyl-1-phenylpropyl]-2,6-dimethylbenzamid chiral
(±)-N-[2-(Dimethylamino)-2-methyl-1-phenylpropyl]-2-methylbenzamid
(±)-2,4-Dichlor-N-[2-(dimethylamino)-2-methyl-1-phenylpropyl]benzamid
(±)-2-Chlor-N-[2-(dimethylamino)-2-methyl-1-phenylpropyl]benzamid
(±)-N-[2-(Dimethylamino)-2-methyl-1-phenylpropyl]-2,5-bis(trifluormethyl)benzamid
N-[2-(Dimethylamino)-2-methyl-1-phenylpropyl]-2-methyl-6-(methyloxy)benzamid chiral
(±)-N-[2-Dimethylamino)-2-methyl-1-phenylpropyl]-2-methyl-3-(trifluormethyl)-benzamid
N-[2-(Dimethylamino)-2-methyl-1-phenylpropyl]-2-(ethyloxy)-4,6-bis(trifluormethyl)-benzamid chiral
(±)-3-Chlor-N-[2-(dimethylamino)-2-methyl-1-phenylpropyl]-2-methylbenzamid
(±)-2-Chlor-N-[2-(dimethylamino)-2-methyl-1-phenylpropyl]-3-methylbenzamid
(±)-N-[2-(Dimethylamino)-2-methyl-1-phenylpropyl]-2,5-dimethylbenzamid
(±)-N-[2-(Dimethylamino)-2-methyl-1-phenylpropyl]-5-fluor-2-methylbenzamid
N-[2-(Dimethylamino)-2-methyl-1-phenylpropyl]-5-fluor-2-(trifluormethyl)benzamid chiral
2,3-Dichlor-N-[2-(dimethylamino)-2-methyl-1-phenylpropyl]-benzamid chiral
2-Chlor-N-[2-(dimethylamino)-2-methyl-1-phenylpropyl]-4-fluorbenzamid chiral
2,4-Dichlor-N-[2-(dimethylamino)-2-methyl-1-phenylpropyl]-5-fluorbenzamid chiral
N-[2-(Dimethylamino)-2-methyl-1-phenylpropyl]-2,4-bis(trifluormethyl)benzamid chiral
(±)-2-Brom-N-[2-(dimethylamino)-2-methyl-1-phenylpropyl]benzamid
(±)-2-Brom-N-[2-(dimethylamino)-2-methyl-1-phenylpropyl]-3-fluorbenzamid
(±)-N-[2-(Dimethylamino)-2-methyl-1-phenylpropyl]-2-iodbenzamid
(±)-N-[2-(Dimethylamino)-2-methyl-1-phenylpropyl]-4-fluor-2-(trifluormethyl)benzamid
(±)-N-[2-(Dimethylamino)-2-methyl-1-phenylpropyl]-2-(methylthio)benzamid
(±)-2-Chlor-N-[2-(dimethylamino)-2-methyl-1-phenylpropyl]-5-(methyloxy)benzamid
(±)-N-[2-(Dimethylamino)-2-methyl-1-phenylpropyl]-2-methyl-4-(methyloxy)benzamid
(±)-2-Chlor-N-[2-(dimethylamino)-2-methyl-1-phenylpropyl]-5-methylbenzamid
2,6-Dichlor-N-[2-(dimethylamino)-2-methyl-1-phenylpropyl]-3-(trifluormethyl)benzamid chiral
(±)-2-Chlor-N-[2-(dimethylamino)-2-methyl-1-phenylpropyl]-5-(trifluormethyl)benzamid
(±)-N-[2-(Dimethylamino)-2-methyl-1-phenylpropyl]-3-fluor-2-methylbenzamid
(±)-N-[2-(Dimethylamino)-2-methyl-1-phenylpropyl]-2,4-dimethylbenzamid
4-Chlor-N-[2-(dimethylamino)-2-methyl-1-phenylpropyl]-2-methyl-6-(methylthio)-benzamid chiral
(±)-2,6-Dichlor-N-[2-(dimethylamino)-2-methyl-1-phenylpropyl]benzamid
(±)-2-Chlor-N-[2-(dimethylamino)-2-methyl-1-phenylpropyl]-3-(trifluormethyl)benzamid
N-[2-(Dimethylamino)-2-methyl-1-phenylpropyl]-2-methyl-4,6-bis(trifluormethyl)-benzamid chiral
ist oder ein Salz oder ein Solvat hiervon.

4. Verbindung gemäß einem der Ansprüche 1 bis 3 zur Verwendung in der Therapie.

5. Verbindung gemäß Anspruch 4 zur Verwendung bei der Behandlung einer durch GlyT1 vermittelten Störung.

6. Verbindung gemäß Anspruch 5, wobei die Störung eine Psychose ist, einschließlich Schizophrenie, Demenz oder Aufinerksamkeitsdefizitsyndrom.

7. Verwendung einer wie in Anspruch 1 beanspruchten Verbindung zur Herstellung eines Medikaments zur Behandlung einer durch GlyT1 vermittelten Störung.

8. Verwendung gemäß Anspruch 7, wobei die Störung eine Psychose ist, einschließlich Schizophrenie, Demenz oder Aufmerksamkeitsdefizitsyndrom.

9. Arzneimittel, das eine Verbindung gemäß Anspruch 4 und mindestens einen pharmazeutisch verträglichen Träger, ein Verdünnungsmittel oder einen Exzipienten umfasst.

10. Arzneimittel gemäß Anspruch 9, das weiter ein oder mehrere therapeutische Mittel umfasst, ausgewählt aus Antidepressiva, die ausgewählt sind aus 5HT3 Antagonisten, Serotoninagonisten, NK-1 Antagonisten, selektiven Serotonin-Wiederaufnahmehemmem (SSRI), Noradrenalin-Wiederaufnahmehemmern (SNRI), trizyklischen Antidepressiva, dopaminergenen Antidepressiva, H3 Antagonisten, 5HT1A Antagonisten, 5HT1B Antagonisten, 5HT1D Antagonisten, D1 Agonisten, M1 Agonisten, krampflösenden Mitteln; atypischen antipsychotischen Arzneistoffen und Wahrnehmungsverstärkern.

11. Verfahren zur Herstellung einer Verbindung wie in Formel (I) in einem der Ansprüche 1 bis 3 definiert, umfassend den Schritt Umsetzen einer Verbindung der Formel (II): 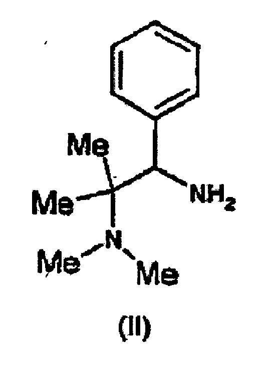 mit einer Verbindung der Formel (III): 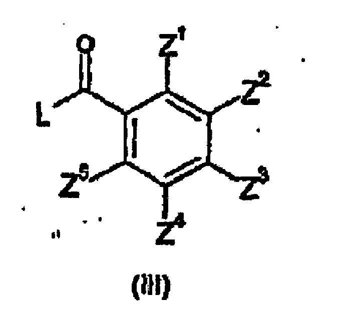 wobei Z¹, Z², Z³, Z⁴ und Z⁵ wie in Formel (I) in einem der Ansprüche 1 bis 3 definiert sind und L eine geeignete Abgangsgruppe darstellt;
und sodann gegebenenfalls:
• Entfernen jeglicher Schutzgruppen und/oder
• Umwandeln einer Verbindung der Formel (I) in eine andere Verbindung der Formel (I) und/oder
• Bilden eines Salzes oder Solvats.

## Revendications

1. Composé de formule (I) ou un de ses sels ou produits de solvatation : 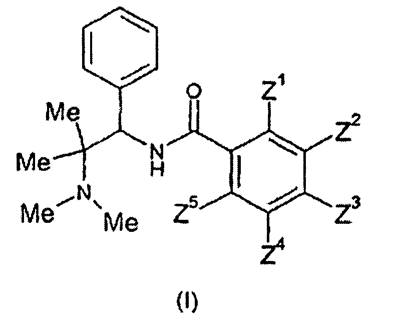 formule dans laquelle
z¹ est choisi dans le groupe consistant en des groupes alkyle en C₁ à C₄, cycloalkyle en C₃ à C₆, alkylthio en C₁ à C₄, halogénalkyle en C₁ à C₄, phényle, halogénophényle, alkylsulfoxy en C₁ à C₄, alkylsulfonyle en C₁ à C₄, chloro, bromo et iodo ;
Z² est choisi dans le groupe consistant en un atome d'hydrogène, un atome d'halogène, des groupes alkyle en C₁ à C₄, phényle, halogénalkyle en C₁ à C₄ , halogénalkoxy en C₁ à C₄, halogénophényle, (alkoxy en C₁ à C₄) (alkyle en C₁ à C₄) et cycloalkyle en C₃ à C₆ ;
Z³ est choisi dans le groupe consistant en un atome d'hydrogène, un atome d'halogène, des groupes alkyle en C₁ à C₄, alkoxy en C₁ à C₄, alkylthio en C₁ à C₄, halogénalkyle en C₁ à C₄, halogénalkoxy en C₁ à C₄ et cycloalkyle en C₃ à C₆ ;
Z⁴ est choisi dans le groupe consistant en un atome d'hydrogène, un atome d'halogène, des groupes alkyle en C₁ à C₄ , halogénalkyle en C₁ à C₄ , alkoxy en C₁ à C₄ , alkylthio en C₁ à C₄, phényle, halogénalkoxy en C₁ à C₄, halogénophényle, (alkoxy en C₁ à C₄) (alkyle en C₁ à C₄) et cycloalkyle en C₃ à C₆ ;
z⁵ est choisi dans le groupe consistant en un atome d'hydrogène, des groupes chloro, bromo, iodo, hydroxy, alkyle en C₁ à C₄ , alkoxy en C₁ à C₄, alkylthio en C₁ à C₄ , phényle, halogénalkoxy en C₁ à C₄, halogénophényle, (alkoxy en C₁ à C₄) (alkyle en C₁ à C₄) et cycloalkyle en C₃ à C₆.

2. Composé suivant la revendication 1, qui est un composé de formule (Ia) ou un de ses sels ou produits de solvatation : 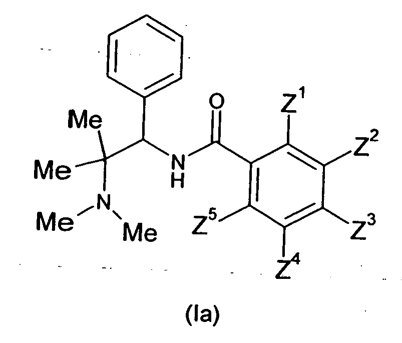 formule dans laquelle
z¹ est choisi dans le groupe consistant en des groupes alkyle en C₁ à C₄, alkylthio en C₁ à C₄, halogénalkyle en C₁ à C₄, alkylsulfoxy en C₁ à C₄, chloro et bromo ;
Z² est choisi dans le groupe consistant en un atome d'hydrogène, un atome d'halogène, des groupes alkyle en C1 à C₄ et halogénalkyle en C₁ à C₄ ;
Z³ est choisi dans le groupe consistant en un atome d'hydrogène, un atome d'halogène, des groupes alkyle en C₁ à C₄, alkoxy en C₁ à C₄ et halogénalkyle en C₁ à C₄ ;
z⁴ est choisi dans le groupe consistant en un atome d'hydrogène, un atome d'halogène, des groupes alkyle en C₁ à C₄, alkoxy en C₁ à C₄ et halogénalkyle en C₁ à C₄ ;
Z⁵ est choisi dans le groupe consistant en un atome d'hydrogène, des groupes chloro, alkyle en C₁ à C₄ et alkoxy en C₁ à C₄.

3. Composé suivant la revendication 1 ou la revendication 2, qui est :
le (+)-4-chloro-N-[2-(diméthylamino)-2-méthyl-1-phényl-propyl]-2-méthyl-6-(méthylthio)benzamide ;
le (+)-N-[2-(diméthylamino)-2-méthyl-1-phénylpropyl]-2-méthyl-6-(méthyloxy)benzamide ;
le *N*-[2-(diméthylamino)-2-méthyl-1-phénylpropyl]-2,6-diméthylbenzamide chiral ;
le 4-chloro-*N*-[2-(diméthylamino)-2-méthyl-1-phénylpropyl]-2,6-diméthylbenzamide chiral ;
le *N*-[2-(diméthylamino)-2-méthyl-1-phénylpropyl]-2-(méthyl-oxy)-4,6-bis(trifluorométhyl)benzamide chiral ;
le (±)-*N*-[2-(diméthylamino)-2-méthyl-1-phénylpropyl]-2,3-diméthylbenzamide ;
le (+)-*N*-[2-(diméthylamino)-2-méthyl-1-phénylpropyl]-2,6-diméthylbenzamide ;
le (+)-*N*-[2-(diméthylamino)-2-méthyl-1-phénylpropyl]-2-méthyl-5-(trifluorométhyl)benzamide ;
le 3-bromo-*N*-[2-(diméthylamino)-2-méthyl-1-phénylpropyl]-2-méthylbenzamide chiral ;
le *N*-[2-(diméthylamino)-2-méthyl-1-phénylpropyl]-2,3-diméthylbenzamide chiral ;
le *N*-[2-(diméthylamino)-2-méthyl-1-phénylpropyl]-2,4,6-triméthylbenzamide chiral ;
le 2-bromo-*N*-[2-(diméthylamino)-2-méthyl-1-phénylpropyl]-6-méthylbenzamide chiral ;
le 3-chloro-*N*-[2-(diméthylamino)-2-méthyl-1-phénylpropyl]-2-méthylbenzamide chiral ;
le (+)-2-bromo-*N*-[2-(diméthylamino)-2-méthyl-1-phényl-propyl]-6-méthylbenzamide ;
le 2-chloro-*N*-[2-(diméthylamino)-2-méthyl-1-phénylpropyl]-6-méthylbenzamide chiral ;
le (±)-2-chloro-*N*-[2-(diméthylamino)-2-méthyl-1-phényl-propyl]-6-méthylbenzamide ;
le 4-chloro-*N*-[2-(diméthylamino)-2-méthyl-1-phénylpropyl]-2-méthyl-6-(méthylsulfinyl)benzamide chiral ;
le 2,4,6-trichloro-*N*-[2-(diméthylamino)-2-méthyl-1-phényl-propyl]benzamide chiral ;
le 2-chloro-*N*-[2-(diméthylamino)-2-méthyl-1-phénylpropyl]-6-(méthyloxy)benzamide chiral ;
le (+)-2-chloro-*N*-[2-(diméthylamino)-2-méthyl-1-phényl-propyl]-6-(méthyloxy)benzamide ;
le (±)-2-bromo-6-chloro-*N*-[2-(diméthylamino)-2-méthyl-1-phénylpropyl]benzamide ;
le (±)-*N*-[2-(diméthylamino)-2-méthyl-1-phénylpropyl]-2,6-diméthyl-4-(méthyloxy)benzamide ;
le *N*-[2-(diméthylamino)-2-méthyl-1-phénylpropyl]-2-propylbenzamide chiral ;
le (±)-3-bromo-*N*-[2-(diméthylamino)-2-méthyl-1-phényl-propyl]-2-méthylbenzamide ;
le (±)-2,4,6-trichloro-*N*-[2-(diméthylamino)-2-méthyl-1-phénylpropyl]benzamide ;
le 5-bromo-*N*-[2-(diméthylamino)-2-méthyl-1-phénylpropyl]-2,3,4-triméthylbenzamide chiral ;
le *N*-[2-(diméthylamino)-2-méthyl-1-phénylpropyl]-2,6-diéthylbenzamide chiral ;
le *N*-[2-(diméthylamino)-2-méthyl-1-phénylpropyl]-2,3,4-triméthylbenzamide chiral ;
le *N*-[2-(diméthylamino)-2-méthyl-1-phénylpropyl]-2,5-diméthyl-4-(méthyloxy)benzamide chiral ;
le *N*-[2-(diméthylamino)-2-méthyl-1-phénylpropyl]-2,3-diméthyl-4-(méthyloxy)benzamide chiral ;
le *N*-[2-(diméthylamino)-2-méthyl-1-phénylpropyl]-2-(1-méthyléthyl)benzamide chiral ;
le (±)-*N*-[2-(diméthylamino)-2-méthyl-1-phénylpropyl]-2,4,6-triméthylbenzamide ;
le 3-chloro-*N*-[2-(diméthylamino)-2-méthyl-1-phénylpropyl]-2,6-diméthylbenzamide chiral ;
le (±)-*N*-[2-(diméthylamino)-2-méthyl-1-phénylpropyl]-2-méthylbenzamide ;
le (±)-2,4-dichloro-N-[2-(diméthylamino)-2-méthyl-1-phényl-propyl]benzamide ;
le (±)-2-chloro-*N*-[2-(diméthylamino)-2-méthyl-1-phényl-propyl]benzamide ;
le (±)-*N*-[2-(diméthylamino)-2-méthyl-1-phénylpropyl]-2,5-bis(trifluorométhyl)benzamide ;
le *N*-[2-(diméthylamino)-2-méthyl-1-phénylpropyl]-2-méthyl-6-(méthyloxy)benzamide chiral ;
le (±)-*N*-[2-(diméthylamino)-2-méthyl-1-phénylpropyl]-2-méthyl-3-(trifluorométhyl)benzamide ;
le *N*-[2-(diméthylamino)-2-méthyl-1-phénylpropyl]-2-(éthyl-oxy)-4,6-bis(trifluorométhyl)benzamide chiral ;
le (+)-3-chloro-*N*-[2-(diméthylamino)-2-méthyl-1-phényl-propyl]-2-méthylbenzamide ;
le (±)-2-chloro-*N*-[2-(diméthylamino)-2-méthyl-1-phényl-propyl]-3-méthylbenzamide ;
le (±)-*N*-[2-(diméthylamino)-2-méthyl-1-phénylpropyl]-2,5-diméthylbenzamide ;
le (±)-*N*-[2-(diméthylamino)-2-méthyl-1-phénylpropyl]-5-fluoro-2-méthylbenzamide ;
le *N*-[2-(diméthylamino)-2-méthyl-1-phénylpropyl]-5-fluoro-2-(trifluorométhyl)benzamide chiral ;
le 2,3-dichloro-*N*-[2-(diméthylamino)-2-méthyl-1-phényl-propyl]benzamide chiral ;
le 2-chloro-*N*-[2-(diméthylamino)-2-méthyl-1-phénylpropyl]-4-fluorobenzamide chiral ;
le 2,4-dichloro-*N*-[2-(diméthylamino)-2-méthyl-1-phényl-propyl]-5-fluorobenzamide chiral ;
le *N*-[2-(diméthylamino)-2-méthyl-1-phénylpropyl]-2,4-bis(trifluorométhyl)benzamide chiral ;
le (+)-2-bromo-*N*-[2-(diméthylamino)-2-méthyl-1-phényl-propyl]benzamide ;
le (+)-2-bromo-*N*-[2-(diméthylamino)-2-méthyl-1-phényl-propyl]-3-fluorobenzamide ;
le (±)-*N*-[2-(diméthylamino)-2-méthyl-1-phénylpropyl]-2-iodobenzamide ;
le (±)-*N*-[2-(diméthylamino)-2-méthyl-1-phénylpropyl]-4-fluoro-2-(trifluorométhyl)benzamide ;
le (±)-*N*-[2-(diméthylamino)-2-méthyl-1-phénylpropyl]-2-(méthylthio)benzamide ;
le (±)-2-chloro-*N*-[2-(diméthylamino)-2-méthyl-1-phényl-propyl]-5-(méthyloxy)benzamide ;
le (±)-*N*-[2-(diméthylamino)-2-méthyl-1-phénylpropyl]-2-méthyl-4-(méthyloxy)benzamide ;
le (+)-2-chloro-*N*-[2-(diméthylamino)-2-méthyl-1-phényl-propyl]-5-méthylbenzamide ;
le 2,6-dichloro-*N*-[2-(diméthylamino)-2-méthyl-1-phényl-propyl]-3-(trifluorométhyl)benzamide chiral ;
le (+)-2-chloro-*N*-[2-(diméthylamino)-2-méthyl-1-phényl-propyl]-5-(trifluorométhyl)benzamide ;
le (±)-*N*-[2-(diméthylamino)-2-méthyl-1-phénylpropyl]-3-fluoro-2-méthylbenzamide ;
le (±)-*N*-[2-(diméthylamino)-2-méthyl-1-phénylpropyl]-2,4-diméthylbenzamide ;
le 4-chloro-*N*-[2-(diméthylamino)-2-méthyl-1-phénylpropyl]-2-méthyl-6-(méthylthio)benzamide chiral ;
le (±)-2,6-dichloro-*N*-[2-(diméthylamino)-2-méthyl-1-phényl-propyl]benzamide ;
le (+)-2-chloro-*N*-[2-(diméthylamino)-2-méthyl-1-phényl-propyl]-3-(trifluorométhyl)benzamide ;
le *N*-[2-(diméthylamino)-2-méthyl-1-phénylpropyl]-2-méthyl-4,6-bis(trifluorométhyl)benzamide chiral ;
ou un de ses sels ou produits de solvatation.

4. Composé suivant l'une quelconque des revendications 1 à 3, destiné à être utilisé en thérapie.

5. Composé suivant la revendication 4, destiné à être utilisé dans le traitement d'un trouble à médiation par GlyT1.

6. Composé suivant la revendication 5, dans lequel le trouble est une psychose, comprenant la schizophrénie, la démence ou le trouble de déficit d'attention.

7. Utilisation d'un composé suivant la revendication 1 dans la préparation d'un médicament destiné au traitement d'un trouble à médiation par GlyT1.

8. Utilisation suivant la revendication 7, dans laquelle le trouble est une psychose, comprenant la schizophrénie, la démence ou le trouble de déficit d'attention.

9. Composition pharmaceutique comprenant un composé suivant la revendication 4, et au moins un support, diluant ou excipient pharmaceutiquement acceptable.

10. Composition pharmaceutique suivant la revendication 9, comprenant en outre un ou plusieurs autres agents thérapeutiques, choisis entre des agents antidépresseurs choisis entre des antagonistes de 5HT3, des agonistes de sérotonine, des antagonistes de NK-1, des inhibiteurs de réabsorption de sérotonine sélectifs (SSRI), des inhibiteurs de réabsorption de noradrénaline (SNRI), des antidépresseurs tricycliques, des antidépresseurs dopaminergiques, des antagonistes de H3, des antagonistes de 5HT1A, des antagonistes de 5HT1B, des antagonistes de 5HT1D, des agonistes de D1, des agonistes de M1, des agents anticonvulsivants, des médicaments antipsychotiques atypiques et des agents améliorant la cognition.

11. Procédé pour la préparation d'un composé tel que défini pour la formule (I) dans l'une quelconque des revendications 1 à 3, comprenant l'étape de réaction d'un composé de formule (II) : 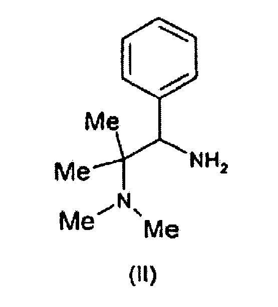 avec un composé de formule (III) 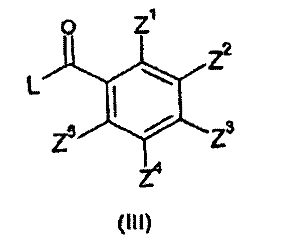 dans laquelle Z¹, Z², Z³, Z⁴ et Z⁵ sont tels que définis pour la formule (I) dans l'une quelconque des revendications 1 à 3 et L représente un groupe partant convenable ; et ensuite facultativement :
• l'élimination de n'importe quels groupes protecteurs et/ou
• la conversion d'un composé de formule (I) en un autre composé de formule (I), et/ou
• la formation d'un sel ou produit de solvatation.
